(19) Europäisches Patentamt
European Patent Office
Office européen des brevets

(11) EP 3 520 886 B1

# (12) EUROPEAN PATENT SPECIFICATION

(45) Date of publication and mention of the grant of the patent:
**19.11.2025 Bulletin 2025/47**

(21) Application number: **17856400.1**

(22) Date of filing: **29.09.2017**

(51) International Patent Classification (IPC):
***B01D 69/02*** (2006.01) ***A61M 1/18*** (2006.01)
***B01D 71/44*** (2006.01) ***B01D 71/68*** (2006.01)

(52) Cooperative Patent Classification (CPC):
**B01D 69/02; A61M 1/1621; B01D 63/031; B01D 69/081; B01D 71/4011; B01D 71/441; B01D 71/68; B01D 71/76;** A61M 1/3623; B01D 2325/04; B01D 2325/20

(86) International application number:
**PCT/JP2017/035373**

(87) International publication number:
**WO 2018/062451 (05.04.2018 Gazette 2018/14)**

## (54) SEPARATION MEMBRANE MODULE

TRENNMEMBRANMODUL

MODULE DE MEMBRANE DE SÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2016 JP 2016193596**

(43) Date of publication of application:
**07.08.2019 Bulletin 2019/32**

(73) Proprietor: **Toray Industries, Inc.**
**Tokyo 103-8666 (JP)**

(72) Inventors:
- **USHIRO, Suguru**
  **Otsu-shi**
  **Shiga 520-8558 (JP)**
- **HAYASHI, Akihiro**
  **Otsu-shi**
  **Shiga 520-8558 (JP)**
- **UENO, Yoshiyuki**
  **Otsu-shi**
  **Shiga 520-8558 (JP)**

(74) Representative: **Kador & Partner Part mbB**
**Corneliusstraße 15**
**80469 München (DE)**

(56) References cited:
**EP-A1- 2 737 916**
**EP-A1- 3 279 227**
**EP-A1- 3 495 004**
**EP-A1- 3 495 398**
**EP-A1- 3 508 232**
**WO-A1-2016/158388**
**DE-A1- 102004 008 220**
**JP-A- 2003 010 322**
**JP-A- 2006 288 866**
**JP-A- 2011 072 987**
**JP-A- H02 229 839**
**JP-A- H07 289 863**
**JP-A- H09 313 906**
**JP-A- S 509 673**
**JP-A- S49 125 493**
**JP-A- S62 109 575**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 520 886 B1

**Description**

TECHNICAL FIELD

The present invention relates to a separation membrane module.

BACKGROUND ART

A medical separation membrane that comes into contact with body fluid or blood has a serious problem that the performance of the medical separation membrane is deteriorated due to adherence of platelets or proteins and the medical separation membrane may cause biological reaction. In particular, a continuous blood purification device used for treatment of acute renal failure is required to be used continuously for one day to several days, and thus it is important that the purification device have specifications that suppress the adherence of platelets or proteins and be capable of withstanding long-term use. Water treatment membranes and biological component separation membranes such as water purifier membranes, water purification membranes, sewage purification membranes, and reverse osmosis membranes are also required to be used continuously for one day to several days, and it is known that the adherence of proteins or organic compounds cause the deterioration in performance of the separation membrane. So far, attempts to solve such a problem by rendering the surface of medical materials hydrophilic have been made, and various investigations have been made.

Patent Document 1 discloses a polysulfone-based polymer that imparts hydrophilicity to a membrane and inhibits adherence of fouling by adding polyvinylpyrrolidone, which is a hydrophilic polymer, at the stage of a membrane forming dope solution and forming the mixture.

Patent Document 2 discloses a separation membrane of a polysulfone-based polymer in which a coating layer insolubilized by radiation crosslinking is formed after contact with a solution of a hydrophilic polymer such as polyvinylpyrrolidone.

Patent Documents 3 and 4 disclose a separation membrane of a polysulfone-based polymer having a vinylpyrrolidone/vinyl acetate copolymer fixed on the surface.

Patent Document 5 discloses a method of efficiently forming a coating layer on a membrane surface by hydrophobic interaction between polysulfone and vinyl acetate that is produced by contacting a polyvinyl alcohol aqueous solution having a saponification degree within a certain range with a polysulfone-based separation membrane.

Membranes modified with a copolymer comprising hydrophilic and hydrophobic units are also disclosed in EP 2 737 916.

PRIOR ART DOCUMENTS

PATENT DOCUMENTS

Patent Document 1: Japanese Published Examined Application No. 02-18695
Patent Document 2: Japanese Patent Laid-open Publication No. 06-238139
Patent Document 3: Japanese Patent Laid-open Publication No. 2010-104984
Patent Document 4: Japanese Patent Laid-open Publication No. 2011-173115
Patent Document 5: Japanese Patent Laid-open Publication No. 2006-198611

SUMMARY OF THE INVENTION

PROBLEMS TO BE SOLVED BY THE INVENTION

However, in the methods described in Patent Documents 1 and 2, a coating layer cannot be formed because of weak interaction between a hydrophilic polymer such as polyvinylpyrrolidone and a polysulfone-based polymer that is a hydrophobic polymer. Therefore, in these methods, to impart hydrophilicity to the surface, many hydrophilic polymers in the membrane forming dope solution need to be used, and the hydrophilic polymers need to be limited to hydrophilic polymer having compatibility with the base polymer.

Meanwhile, in the methods described in Patent Documents 3 and 4, a vinyl acetate unit interacts with a hydrophobic base, whereby the introduction efficiency of the copolymer is increased, and the hydrophilization can be efficiently performed.

However, in the methods described in Patent Documents 3 and 4, a vinylpyrrolidone/vinyl acetate copolymer,

which is a commercially available polymer, is used, and no structural design suitable for suppressing the adherence of platelets or proteins is considered. In fact, the present inventors produced a medical material according to the methods described in Patent Documents 3 and 4, and proved that when the medical material is in contact with biological components such as blood for a long time, platelets or proteins adhere to the medical material and the performance of the medical material is deteriorated. Further, it was found that when the introduction amount of the copolymer is increased in order to suppress the adherence of platelets or proteins, the water removal performance is deteriorated and the amount of the eluate is increased.

The present inventors investigated the method described in Patent Document 5, and found that when the separation membrane is coated with polyvinyl alcohol, the performance of the separation membrane is remarkably deteriorated. Further, it is also known that hydroxyl groups of polyvinyl alcohol and the like tend to activate complements when in contact with blood.

Therefore, an object of the present invention is to provide a separation membrane module that has little deterioration in performance over time even when in contact with biological components such as blood for a long time, is excellent in removal performance of water and the like, and has little amount of the eluate.

SOLUTIONS TO THE PROBLEMS

Since a protein contained in biological components such as blood tends to adhere to a hydrophobic surface, it is important that the entire contact surface of a medical material have hydrophilicity. It is thought that this is because when the protein approaches the surface of the material, the conformation of the protein changes, the hydrophobic site inside the protein is exposed, and the hydrophobic site interacts with the material surface.

Meanwhile, it is known that the adherence of proteins and the like cannot be suppressed when the contact surface of the medical material is coated with a hydrophilic polymer such as polyethylene glycol or polyvinyl alcohol. It is thought that this is because when the hydrophilicity of the contact surface of the medical material is too strong, the structure of the protein becomes unstable, and thus the adherence of the protein cannot be sufficiently suppressed.

As a result of intensive studies to solve the above problems, the present inventors have found the following separation membrane module having greatly suppressed adherence of platelets or proteins, and no deterioration in performance even when in contact with biological components such as blood for a long time.

Claimed is a separation membrane module including a separation membrane comprising a hydrophobic polymer, a hydrophilic polymer, and polymer A, wherein the polymer A comprises a hydrophilic unit and a hydrophobic unit, and is a copolymer wherein the hydrophobic unit is a propyl methacrylate unit, the separation membrane module having a retention rate of an albumin sieving coefficient of 86% or more at 60 minutes after circulation start relative to an albumin sieving coefficient at 10 minutes after circulation start when 2 L of bovine blood containing 50 U/ml of heparin, and having a hematocrit of 30% by volume and a total protein concentration of 6 to 7 g/dl is circulated at a flow rate of 100 ml/min at 37°C and a filtration flow rate of 10 ml/(min·m$^2$).

In the separation membrane module, the separation membrane preferably includes a swelling layer having a thickness of 9 to 50 nm on an inner surface of the separation membrane, and the inner surface of the separation membrane has a number of an adhered human platelet of 10/4.3 $\times$ 10$^3$ $\mu$m$^2$ or less.

In the separation membrane module, the separation membrane is preferably a hollow fiber membrane having an inner diameter of 100 to 400 $\mu$m and a membrane thickness of 10 to 60 $\mu$m.

In the separation membrane module, the hollow fiber membrane preferably has a total of an inner surface area of 0.3 to 3.0 m$^2$.

In the separation membrane module, the hydrophilic unit preferably is a vinylpyrrolidone unit, an N-vinylacetamide derivative unit, an acrylamide derivative unit, or a methacrylamide derivative unit.

In the separation membrane module, the hydrophobic polymer preferably is a polysulfone-based polymer, and the hydrophilic polymer is polyvinylpyrrolidone. The separation membrane module is for blood purification.

EFFECTS OF THE INVENTION

The separation membrane module of the present invention has suppressed adherence of platelets or proteins, has little deterioration in performance even when used for a long time, and can be used as a separation membrane module for blood purification.

BRIEF DESCRIPTION OF THE DRAWINGS

Fig. 1 shows a schematic diagram of a section horizontal to the longitudinal direction of a hollow fiber membrane

module which is one of the forms of a separation membrane module.

Fig. 2 shows a schematic diagram of an instrument and a circuit for measuring the retention rate of an albumin sieving coefficient.

Fig. 3 shows a schematic diagram of a force curve of an atomic force microscope.

Fig. 4 shows a schematic diagram of a section horizontal to the short direction of a hollow fiber membrane.

EMBODIMENTS OF THE INVENTION

Hereinafter, the present invention will be described in detail.

A separation membrane module of the present invention includes a separation membrane comprising a hydrophobic polymer, a hydrophilic polymer, and polymer A, wherein the polymer A comprises a hydrophilic unit and a hydrophobic unit, and is a copolymer wherein the hydrophobic unit is a propyl methacrylate unit, the separation membrane module having a retention rate of an albumin sieving coefficient of 86% or more at 60 minutes after circulation start relative to an albumin sieving coefficient at 10 minutes after circulation start when 2 L of bovine blood containing 50 U/ml of heparin, and having a hematocrit of 30% by volume and a total protein concentration of 6 to 7 g/dl is circulated at a flow rate of 100 ml/min at 37°C and a filtration flow rate of 10 ml/(min·$m^2$).

The "separation membrane" is a membrane that selectively removes a specific substance contained in a liquid to be treated such as blood or an aqueous solution by adsorption or based on the size and the like of the substance.

The "separation membrane module" is a device in which the separation membrane is incorporated.

The separation membrane comprises a hydrophobic polymer, a hydrophilic polymer, and polymer A. The hydrophobic polymer plays a role of imparting strength so that the shape of the separation membrane can be retained even when the separation membrane is in contact with biological components such as blood for a long time. The hydrophilic polymer has a role of imparting hydrophilicity to the interior of the separation membrane and forming small holes through which substances to be removed such as water and waste products pass. The polymer A has a role of suppressing the adherence of platelets or proteins and may be provided to the entire separation membrane. However, from the viewpoint of cost, it is preferably provided to at least the surface of the separation membrane that comes into in contact with blood.

The "hydrophobic polymer" refers to a polymer having a solubility of 1 g or less in 100 g of pure water at 20°C when the number average molecular weight of the polymer is 1,000 or more and 50,000 or less, and the hydrophobic polymer preferably has the solubility of 0.1 g or less, more preferably has the solubility of 0.01 g or less.

Examples of the hydrophobic polymer include, without particular limitation, hydrophobic polymers such as a polysulfone-based polymer, polystyrene, polyurethane, polyethylene, polypropylene, polycarbonate, polyvinylidene fluoride, polyacrylonitrile, polymethyl methacrylate, polyvinyl chloride, and polyester. Among these, the polysulfone-based polymer and polymethyl methacrylate are preferably used because they are easily formed into a separation membrane. The hydrophobic polymer can be purchased or can be produced by a known method or a method similar to the known method.

The "hydrophilic polymer" refers to a polymer having a solubility of 1 g or more in 100 g of pure water at 20°C when the number average molecular weight of the polymer is 1,000 or more and 50,000 or less, and the hydrophilic polymer preferably has the solubility of 10 g or more.

Examples of the hydrophilic polymer include polyethylene glycol, polyvinyl alcohol, polyvinylpyrrolidone, carboxymethyl cellulose, and polypropylene glycol. The hydrophilic polymer is preferably at least one hydrophilic polymer selected from the group consisting of polyvinylpyrrolidone, polyethylene glycol, and polyvinyl alcohol. Among these, when a polysulfone-based polymer is used as the hydrophobic polymer, polyvinylpyrrolidone is preferably used from the viewpoint of compatibility and safety. The hydrophilic polymer can be purchased or can be produced by a known method or a method similar to the known method. Examples of the combination of the hydrophobic polymer and the hydrophilic polymer include a combination of a polysulfone-based polymer as the hydrophobic polymer and polyvinylpyrrolidone as the hydrophilic polymer.

The polymer A comprises a hydrophilic unit and a hydrophobic unit, is a copolymer wherein the hydrophobic unit is a propyl methacrylate unit, and can be produced by a known method or a method similar to the known method.

Examples of the sequence of units in the above copolymer include a block copolymer, an alternating copolymer, and a random copolymer. Among these, the alternating copolymer or the random copolymer is preferred from the viewpoint of small unevenness in hydrophilicity/hydrophobicity and moving properties in the whole copolymer. Among these, the random copolymer is more preferable from the viewpoint that the synthesis is not complex. The copolymer in which at least a part of the monomer sequence is arranged at random is a random copolymer.

The "unit" refers to a repeating unit in a homopolymer or a copolymer obtained by polymerization of monomers. For example, the "hydrophobic unit" refers to a repeating unit in a homopolymer obtained by polymerization of a hydrophobic monomer or a repeating unit derived from a hydrophobic monomer in a copolymer obtained by copolymerization of the hydrophobic monomer.

The "hydrophobic unit" is defined as a repeating unit whose homopolymer (the number average molecular weight

is 1,000 or more and 50,000 or less) is poorly-soluble or insoluble in water. The "poorly-soluble or insoluble in water" refers to a solubility of 1 g or less in 100 g of pure water at 20°C.

The "hydrophilic unit" is defined as a repeating unit whose homopolymer (the number average molecular weight is 1,000 or more and 50,000 or less) is easily-soluble in water. The "easily-soluble in water" refers to a solubility of more than 1 g in 100 g of pure water at 20°C. The hydrophilic unit preferably has the solubility of 10 g or more.

Examples of the hydrophobic unit which are not covered by the claims include a vinyl propionate unit, a vinyl butyrate unit, a vinyl pivalate unit, a vinyl pentanoate unit, a vinyl octanoate unit, a vinyl 2-ethylhexanoate unit, a vinyl stearate unit, an ethyl acrylate unit, a propyl acrylate unit, a butyl acrylate unit, an isobutyl acrylate unit, a tert-butyl acrylate unit, an octyl acrylate unit, a hexadecyl acrylate unit, an ethyl methacrylate unit, a butyl methacrylate unit, an isobutyl methacrylate unit or a tert-butyl methacrylate unit, a tridecyl methacrylate unit, and a 1-butene unit or a 1-nonene unit.

The hydrophobic unit is propyl methacrylate.

Examples of the hydrophilic unit include, without particular limitation, a methacrylic acid unit, an acrylic acid unit, an acrylamide derivative unit, a methacrylamide derivative unit, a 2-hydroxyethyl methacrylate unit, a 2-hydroxyethyl acrylate unit, an N-vinylacetamide derivative unit, a vinylpyrrolidone unit, a vinyl caprolactam unit, a vinyl alcohol unit, and an ethylene glycol unit. Among these, a unit having an amide group is preferable because the hydrophilicity is not too strong. The unit having an amide group may be a unit having an acyclic amide group such as an N-vinylacetamide derivative unit, an acrylamide derivative unit, and a methacrylamide derivative unit, or may be a unit having a cyclic amide group such as a vinylpyrrolidone unit and a vinyl caprolactam unit. However, the vinylpyrrolidone unit, the N-vinylacetamide derivative unit, the acrylamide derivative unit, or the methacrylamide derivative unit is more preferable.

The N-vinylacetamide derivative unit is a unit having a vinylacetamide structure ($CH_2$=CH-NH-CO-), and examples of the N-vinylacetamide derivative unit include an N-vinylacetamide unit, and an N-methyl-N-vinylacetamide unit.

The acrylamide derivative unit is a unit having an acrylamide structure ($CH_2$=CH-CO-NH-), and examples of the acrylamide derivative unit include an acrylamide unit, an N-methyl acrylamide unit, an N-isopropylacrylamide unit, an N-tert-butylacrylamide unit, and an N-phenylacrylamide unit.

The methacrylamide derivative unit is a unit having a methacrylamide structure ($CH_2$=C($CH_3$)-CO-NH-), and examples of the methacrylamide derivative unit include a methacrylamide unit, an N-isopropyl methacrylamide unit, and an N-phenyl methacrylamide unit.

In the copolymer, the mole fraction of the hydrophilic unit in the whole copolymer is preferably 30 to 90%, more preferably 40 to 80%, and further preferably 50 to 70%. Any preferred lower limit can be combined with any preferred upper limit. This is because when the mole fraction of the hydrophilic unit is too small, the hydrophobicity of the whole copolymer becomes strong, and when the molar fraction of the hydrophilic unit is too large, the hydrophilicity of the whole copolymer becomes strong, resulting in an unstable structure of the proteins or platelets. The mole fraction is, for example, calculated from the peak area obtained by nuclear magnetic resonance (NMR) measurement. When the mole fraction cannot be calculated from NMR measurement because of overlapping of the peaks and the like, the mole fraction may be calculated by elementary analysis.

The number average molecular weight of the copolymer is preferably 1,000 or more, more preferably 5,000 or more, because when the number average molecular weight is too small, the effect of suppressing the adherence of platelets or proteins may not be sufficiently exerted. Meanwhile, though the upper limit of the number average molecular weight of the copolymer is not particularly limited, it is preferably 1,000,000 or less, more preferably 500,000 or less, further preferably 100,000 or less because the solubility may decrease when the number average molecular weight is too large. The number average molecular weight of the copolymer can be measured by gel permeation chromatography (GPC) as described below.

In a separation membrane, due to the adherence of proteins or platelets, the fractionation performance and the water permeation performance are deteriorated, and in addition, blood may not pass through the separation membrane due to blood coagulation and the extracorporeal circulation may not be continued. The adherence of platelets or proteins occurs significantly, in particular, within 60 minutes after the contact with blood. Thus, in the present invention, albumin sieving coefficients at 10 minutes after blood circulation start and at 60 minutes after blood circulation start are each measured, and the retention rate is calculated.

When the separation membrane is a hollow fiber membrane, the retention rate of an albumin sieving coefficient is measured as follows. First, a hollow fiber membrane module (21) and a blood circuit are connected as shown in Fig. 2. Citric acid (ACD-A solution, manufactured by TERUMO CORPORATION) (15% by volume) is added to raw blood collected from a bovine for stabilization. Bovine blood containing 50 U/ml of heparin, and having a hematocrit of 30% by volume and a total protein concentration of 6 to 7 g/dl is prepared, and placed in a circulation beaker (24). The circulation beaker (24) containing the bovine blood is kept at 37°C in a warm water bath (29) equipped with a heater (28). The preparation conditions of the bovine blood are based on Japan Industrial Standard JIS T 3250: 2013.

The inlet of a Bi circuit (25), the outlet of a Bo circuit (26), and the outlet of a F circuit (27) are placed in the circulation beaker (24) containing 2 L of the bovine blood prepared above, and a Bi pump (22) is started at a circulation flow

rate of 100 ml/min. The circulation flow rate is the condition generally employed in the treatment with a slow continuous type blood filter.

The Bi circuit (25) refers to the flow path of blood that comes out of the circulation beaker (24), flows through the Bi pump (22), and enters the blood side inlet of the hollow fiber membrane module (21). The Bo circuit (26) refers to the flow path of blood that comes out of the blood side outlet of the hollow fiber membrane module (21) and enters the circulation beaker (24). The F circuit (27) refers to the flow path of blood that comes out of the dialysate side outlet of the hollow fiber membrane module (21), flows through an F pump (23), and enters the circulation beaker (24). The Bi pump (22) refers to a pump used to flow blood through the Bi circuit (25).

Subsequently, the F pump (23) is started at a filtration flow rate of 10 ml/(min·$m^2$), and samples are each collected from the inlet of the Bi circuit (25), the outlet of the Bo circuit (26), and the outlet of the F circuit (27) over time. The F pump (23) refers to a pump used to flow blood through the F circuit (27). The blood sampled at the inlet of the Bi circuit (25) and the outlet of the Bo circuit (26) is centrifuged at 3000 rpm for 10 minutes, then the supernatant plasma is collected and subjected to albumin concentration measurement. The filtration flow rate of 10 ml/(min·$m^2$) refers to filtration at 10 ml/min per 1.0 $m^2$ of the separation membrane. When the separation membrane module has a membrane area of 1.3 $m^2$, filtration is performed at 13 ml/min. The membrane area refers to the area of the separation membrane that is in contact with blood.

The albumin concentration at each elapsed time from the start of the F pump (23) is measured, and the albumin sieving coefficient (ScAlbs) at each elapsed time is calculated by the following formula.

$$ScAlb\ (\%) = 2 \times CF/(CBi + CBo) \times 100$$

In the above formula, CF refers to the albumin concentration (g/ml) at the outlet of the F circuit (27), CBo refers to the albumin concentration (g/ml) at the outlet of the Bo circuit (26), and CBi refers to the albumin concentration (g/ml) at the inlet of the Bi circuit (25).

In the present invention, the retention rate of an albumin sieving coefficient at 60 minutes after circulation start (hereinafter may be referred to as ScAlb60) relative to an albumin sieving coefficient at 10 minutes after circulation start (hereinafter may be referred to as ScAlb10) is calculated by the following formula.

$$Retention\ rate\ (\%) = ScAlb60/ScAlb10 \times 100$$

When the separation membrane is not a hollow fiber membrane, the albumin concentration CBi in the blood flowing into the module, the albumin concentration CBo in the blood flowing out of the module, and the albumin concentration CF in the filtrate are measured, and albumin sieving coefficients are calculated in the same manner as in the hollow fiber membrane.

It is important that the retention rate of an albumin sieving coefficient at 60 minutes after circulation start relative to an albumin sieving coefficient at 10 minutes after circulation start be 86% or more. This is because the adherence of platelets or proteins occurs significantly, in particular, within 60 minutes after the contact with the blood, and thus, the subsequent adherence of platelets or proteins is small, and the membrane can be stably used continuously for 1440 minutes when the retention rate of an albumin sieving coefficient at 60 minutes after circulation start relative to an albumin sieving coefficient at 10 minutes after circulation start is 86% or more. When the separation membrane cannot be used continuously for 1440 minutes, that is, one day, the separation membrane needs to be changed at midnight, which is a burden on medical personnel. The retention rate of an albumin sieving coefficient at 60 minutes after circulation start relative to an albumin sieving coefficient at 10 minutes after circulation start is preferably 90% or more, further preferably 95% or more. It is most preferably 97% or more.

Meanwhile, for the performance after 24 hours (1440 minutes) from the start of use, in the measurement of the retention rate of the albumin sieving coefficient described above, bovine plasma is circulated for 1440 minutes, and the retention rate of an albumin sieving coefficient at 1440 minutes after circulation start (ScAlb1440') relative to an albumin sieving coefficient at 10 minutes after circulation start (ScAlb10') is calculated. When bovine blood is used, bovine plasma having a total protein concentration of 6 to 7 g/dl obtained by centrifugation of bovine blood is used for measurement because the bovine blood may coagulate on the wall or the like of the beaker or cause hemolysis. Heparin (50 U/ml) is added to the bovine plasma.

In the present invention, the retention rate of an albumin sieving coefficient at 1440 minutes after circulation start (hereinafter may be referred to as ScAlb1440') relative to an albumin sieving coefficient at 10 minutes after circulation start (hereinafter may be referred to as ScAlb10') was calculated by the following formula.

$$Retention\ rate\ (\%) = ScAlb1440'/ScAlb10' \times 100$$

The retention rate of an albumin sieving coefficient at 1440 minutes after circulation start relative to an albumin

sieving coefficient at 10 minutes after circulation start is preferably 70% or more, more preferably 80% or more, further preferably 90% or more, most preferably 95% or more for the stable continuous use for 1440 minutes.

In a preferred aspect of the separation membrane, to suppress the adherence of blood components, a separation membrane that comprises the hydrophobic polymer and the hydrophilic polymer, and has the polymer A introduced on the surface thereof (in particular, the inner surface that is often in contact with blood) is preferable. A separation membrane module including the separation membrane incorporated in a casing is preferable. The form of the separation membrane is preferably a hollow fiber membrane, and the separation membrane module is preferably a hollow fiber membrane module.

As a method of introducing the polymer A on the surface of the separation membrane, for example, a method in which a separation membrane is formed, and then coated with the polymer A is preferably used, and a method in which the surface of the separation membrane is brought into contact with a solution (preferably an aqueous solution) of the polymer A is used. More specifically, examples of the method include a method of flowing a solution of a polymer at a predetermined flow rate and a method of immersing the separation membrane in the above solution. In addition, examples of the method include a method of adding the polymer A to the dope solution from which the separation membrane is formed and spinning the mixture under a condition intentionally set so that the polymer gathers on the surface of the separation membrane.

When the aqueous solution in which the polymer A is dissolved is passed through the hollow fiber membrane in the module and introduced into the surface of the separation membrane, a sufficient amount of the polymer is not introduced on the surface if the concentration of the polymer in the aqueous solution is too small. Therefore, the concentration of the polymer in the aqueous solution is preferably 10 ppm or more, more preferably 100 ppm or more, further preferably 300 ppm or more. However, when the concentration of the polymer in the aqueous solution is too large, the eluate from the module may be increased. Thus, the concentration of the polymer in the aqueous solution is preferably 100,000 ppm or less, more preferably 10,000 ppm or less.

When the polymer A does not dissolve in water to a predetermined concentration, the polymer A can be compatibilized with an organic solvent in which the separation membrane dose not dissolve or water, and be dissolved in a mixed solvent of an organic solvent in which the separation membrane dose not dissolve or water. Examples of the organic solvent or the organic solvent used in the mixed solvent include, but are limited to, alcohol solvents such as methanol, ethanol, and propanol.

When the proportion of the organic solvent in the mixed solvent is high, the entire separation membrane may swell and deform, which may result in the decrease of the strength. Therefore, the weight fraction of the organic solvent in the mixed solvent is preferably 60% or less, more preferably 10% or less, further preferably 1% or less.

To prevent the elution in use, it is preferable that the polymer A be introduced on the surface of the separation membrane comprising the hydrophobic polymer and the hydrophilic polymer, and then fixed on the surface of the separation membrane.

As used herein, "fixed on the surface of the separation membrane" refers to chemically or physically bonding the polymer to the surface of the separation membrane by chemical reaction or crosslinking reaction.

In particular, it is preferable from the viewpoint of convenience to introduce the polymer A on the surface of the separation membrane comprising the hydrophobic polymer and the hydrophilic polymer, and then insolubilize and fix the polymer A by radiation or heat treatment.

For the radiation, an $\alpha$ ray, a $\beta$ ray, a $\gamma$ ray, an X ray, an ultraviolet ray, an electron beam or the like can be used. Blood purification devices such as artificial kidneys are obliged to be sterilized prior to shipping, and for the sterilization in recent years, from the viewpoint of the low residual toxicity and convenience, radiation sterilization with a $\gamma$ ray and/or an electron beam is heavily used. Therefore, it is preferable to perform the radiation sterilization in a state where the aqueous solution in which the polymer is dissolved is in contact with the separation membrane because insolubilization of the polymer can be achieved simultaneously with sterilization.

When sterilization and fixation of the separation membrane are performed at the same time, the radiation dose of the ray is preferably 15 kGy or more, more preferably 25 kGy or more. This is because 15 kGy or more is effective for sterilizing a blood purification module and the like with a $\gamma$ ray. The radiation dose is preferably 100 kGy or less. This is because when the radiation dose is more than 100 kGy, the polymer tends to undergo three-dimensional crosslinking, decomposition and the like, and blood compatibility may decrease.

An antioxidant may be used to suppress crosslinking reaction during radiation. The antioxidant refers to a substance having properties of easily giving electrons to other molecules, and examples of the antioxidant includes, but are not limited to, water-soluble vitamins such as vitamin C, polyphenols, and alcohol solvents such as methanol, ethanol, or propanol. These antioxidants may be used alone or in combination of two or more types. When an antioxidant is used in the separation membrane module, safety needs to be considered, and thus, an antioxidant having low toxicity such as ethanol or propanol is preferably used.

Furthermore, as a method of fixing the polymer A on the surface of the separation membrane comprising the hydrophobic polymer and the hydrophilic polymer, bonding by a chemical reaction may be utilized. Specifically, the fixation is achieved by reacting a reactive group such as a hydroxy group, a carboxyl group, an amino group, a sulfonic acid group, a halogenated alkyl group or the like on the surface of the separation membrane, with a reactive group introduced to the

terminal of the main chain or the side-chain of the copolymer.

Examples of the method of introducing a reactive group on the surface of the separation membrane include, for example, a method of polymerizing a monomer having a reactive group to obtain a base having the reactive group on the surface, and a method of introducing a reactive group by ozone treatment, or plasma treatment after polymerization.

Examples of the method of introducing a reactive group to the terminal of the main chain of the polymer A include a method of using an initiator having a reactive group such as 2,2'-azobis [2-methyl-N-(2-hydroxyethyl) propionamide] or 4,4'-azobis (4-cyanovaleric acid).

Examples of the method of introducing a reactive group to the side-chain of the polymer A include a method of copolymerizing a monomer having a reactive group such as glycidyl methacrylate or N-hydroxysuccinimide methacrylate ester to the extent that the action and function of the copolymer are not impaired.

It is preferable that in the separation membrane module of the present invention, the separation membrane include a swelling layer having a thickness of 9 to 50 nm on an inner surface of the separation membrane, and the inner surface of the separation membrane have a number of an adhered human platelet of $10/4.3 \times 10^3\ \mu m^2$ or less.

The "swelling layer" refers to a layer in which the hydrophilic polymer and/or the polymer A present on the inner surface of the separation membrane are swelled by water in a wet state. The wet state means that the water content of the separation membrane is 60% or more. The water content refers to the weight fraction of water in the weight of the whole wetted separation membrane. When the thickness of the swelling layer is too small, the effect of suppressing the adherence of platelets or proteins is reduced. Meanwhile, when the thickness of the swelling layer is too large, small holes of the separation membrane may be blocked, and the water removal performance may be deteriorated. Therefore, the thickness of the swelling layer is preferably 9 to 50 nm, more preferably 10 to 40 nm, and further preferably 10 to 30 nm. Any preferred lower limit can be combined with any preferred upper limit.

The number of an adhered human platelet is a value obtained by calculating the number of the human platelet adhered to the inner surface of the separation membrane as the number per inner surface area of the separation membrane of $4.3 \times 10^3\ \mu m^2$ when the separation membrane is in contact with human blood derived from a healthy human for one hour. The method of measuring the adherence number of a human platelet is as follows. The inner surface of the separation membrane is exposed, brought into contact with human blood derived from a healthy human to which 50 U/ml of heparin is added, and shaken for one hour at 37°C. Thereafter, the separation membrane is washed with saline, blood components are fixed with 2.5% glutaraldehyde saline, and the separation membrane is washed with distilled water and dried under reduced pressure at 20°C and 0.5 Torr for 10 hours. The inner surface of the separation membrane is observed with a field emission type scanning electron microscope at a magnification of 1500 times, and the number of platelets adhered within one field of view ($4.3 \times 10^3\ \mu m^2$) is counted. The average of the numbers of a adhered platelet in 20 different fields of view of the separation membrane is taken as the number of an adhered human platelet (number/$4.3 \times 10^3\ \mu m^2$). When the number of an adhered human platelet is more than $10/4.3 \times 10^3\ \mu m^2$, blood compatibility becomes insufficient, and the effect of suppressing the adherence of organic compounds and biological components such as proteins also becomes insufficient. Thus, the number of an adhered human platelet is more preferably $5/4.3 \times 10^3\ \mu m^2$ or less, further preferably $3/4.3 \times 10^3\ \mu m^2$ or less, most preferably $0/4.3 \times 10^3\ \mu m^2$. The preferable range of the thickness of the swelling layer and the preferable range of the number of an adhered human platelet can be each arbitrarily combined. For example, it is preferable that the separation membrane include a swelling layer having a thickness of 10 to 40 nm on the surface of the separation membrane, and the number of an adhered human platelet be $5/4.3 \times 10^3\ \mu m^2$ or less, and it is more preferable that the separation membrane include a swelling layer having a thickness of 10 to 30 nm on the surface of the separation membrane, and the number of an adhered human platelet be $3/4.3 \times 10^3\ \mu m^2$ or less.

The swelling layer on the inner surface of the separation membrane can be observed using an atomic force microscope (AFM) and the thickness can be calculated from the force curve measurement. As shown in Fig. 3, the force curve is expressed by the displacement amount of the cantilever on the horizontal axis when the vertical axis represents the force applied to the cantilever. The force curve moves parallel to the X axis until the short needle of the cantilever comes into contact with the surface of the separation membrane. After the cantilever comes into contact with the surface of the separation membrane, a curved nonlinear part appears when there is a swelling layer. After the nonlinear part, a linear correlation is obtained between the displacement amount of the cantilever and the force. The swelling layer is defined as a distance (34) from the start of the extension line of the line (31) that moves parallel to the X axis until the short needle of the cantilever comes into contact with the surface to the intersection point of the extension line of a linear part (33) after the curved nonlinear part (32) that appears when the short needle of the cantilever comes into contact with the surface. When the separation membrane is a hollow fiber membrane, the measurement is performed at 5 arbitrarily selected points on the inner surface of a plurality of arbitrarily selected hollow fiber membranes, and the average is taken as the thickness. When the separation membrane is not a hollow fiber membrane, the measurement is also performed at 5 arbitrarily selected points on the inner surface of the separation membrane, and the average is taken as the thickness. The average is rounded off to the nearest whole number.

When the separation membrane is a hollow fiber membrane, the thinner the membrane thickness of the hollow fiber membrane is, the more the film coefficient of mass transfer can be reduced, and thus the substance removal

performance of the separation membrane is improved. Meanwhile, when the membrane thickness of the hollow fiber membrane is too thin, fiber breakage and fiber collapse due to drying tend to occur, which may be a problem in production. Therefore, the membrane thickness of the hollow fiber membrane is preferably 10 to 60 μm, more preferably 20 to 50 μm, further preferably 30 to 45 μm. Any preferred lower limit can be combined with any preferred upper limit.

For the same reason, the inner diameter of the hollow fiber membrane is preferably 100 to 400 μm, more preferably 150 to 300 μm. Any preferred lower limit can be combined with any preferred upper limit. The preferable range of the membrane thickness and the preferable range of the inner diameter of the separation membrane (in particular, the hollow fiber membrane) can be each arbitrarily combined. For example, a hollow fiber membrane having an inner diameter of 100 to 400 μm and a membrane thickness of 10 to 60 μm is preferable, and a hollow fiber membrane having an inner diameter of 150 to 300 μm and a membrane thickness of 20 to 50 μm is more preferable.

Fig. 4 shows a sectional view horizontal to the short direction of a hollow fiber membrane. The membrane thickness of a hollow fiber membrane refers to a thickness (41) of the hollow fiber membrane, and the inner diameter of the hollow fiber membrane refers to a diameter (42) of the cavity of the hollow fiber membrane. The membrane thickness of the hollow fiber membrane can be obtained by measuring the thicknesses of 16 randomly selected hollow fiber membranes with a 1000-fold lens of a microwatcher (VH-2100, manufactured by KEYENCE CORPORATION) and calculating the average. The inner diameter of the hollow fiber membrane can be obtained by measuring outer diameters (43) of 16 randomly selected hollow fiber membranes with a laser displacement meter (for example, LS5040T, manufactured by KEYENCE CORPORATION), calculating the average, and calculating the inner diameter by the following formula.

Inner diameter of hollow fiber membrane (μm) = outer diameter of hollow fiber membrane (μm) - 2 × membrane thickness of hollow fiber membrane (μm)

When the total of the inner surface area of the hollow fiber membrane is too small, the water removal performance may be insufficient. The water removal performance means the capability to remove water from a liquid, in particular blood, flowing inside the hollow fiber membrane. The larger the total of the inner surface area of the hollow fiber membrane is, the larger the contact area with the liquid is, and the water removal performance is increased. Meanwhile, when the total of the inner surface area of the hollow fiber membrane is too large, the hollow fiber membrane module becomes huge, and the handleability is decreased. Therefore, the total of the inner surface area of the hollow fiber membrane is preferably 0.3 to 3.0 $m^2$, more preferably 0.5 to 2.8 $m^2$, and further preferably 0.8 to 2.6 $m^2$. Any preferred lower limit can be combined with any preferred upper limit.

The total of the inner surface area of the hollow fiber membrane is obtained by the following formula.

Total of inner surface area of hollow fiber membrane ($m^2$) = $\pi$ × inner diameter of hollow fiber membrane (m) × effective length (m) × number of hollow fiber (number)

The effective length (m) means the length of the part to which no potting agent is adhered in the hollow fiber membrane placed in the hollow fiber membrane module, and $\pi$ means the circular constant.

When the polymer A is fixed on the surface of the separation membrane, small holes are generally blocked, and the filtration performance, in particular, water permeability of the separation membrane module is significantly deteriorated. Although achieving both high filtration performance and suppression of the adherence of proteins and the like at the same time has been impossible so far, it has been successfully achieved by using the polymer A having an alkyl group having 2 to 20 carbon atoms at a side-chain terminal. It is thought that the interaction with water is weakened due to the alkyl group at a side-chain terminal, and the filtration performance is improved. The separation membrane module is preferably used for, in particular, water treatment and blood purification, and high filtration performance of the separation membrane is required. Thus, the water permeability is preferably 180 ml/hr/mmHg/$m^2$ or more, more preferably 250 ml/hr/mmHg/$m^2$ or more, further preferably 300 ml/hr/mmHg/$m^2$ or more. For the use in blood purification, when the water permeability is too high, a phenomenon such as residual blood may be observed, and thus, the water permeability is preferably 2000 ml/hr/mmHg/$m^2$ or less, more preferably 1500 ml/hr/mmHg/$m^2$ or less. Any preferred lower limit can be combined with any preferred upper limit. The water permeability refers to the water removal performance per inner surface area of the separation membrane.

In the present invention, the water permeability can be obtained as follows. Water (37°C) was flowed through the separation membrane module at 200 ml/min, the outflow at the blood side outlet was adjusted, and the filtration amount V in which the water flows out to the dialysate inlet side per minute, and the average pressure P at the blood side inlet/outlet were measured. Ultrafiltration rate UFR was calculated by the following formula. The outflow from the blood side outlet was changed, measurement was performed at three points, and the average of UFRs was taken as the water permeability of the separation membrane module.

$$UFR\ (ml/hr/mmHg/m^2) = V \times 60/P/A$$

V: filtration amount (ml/min), P: pressure (mmHg), A: membrane area ($m^2$)

Examples of the form of the separation membrane include a laminated type, a coil type, and a hollow fiber type. However, from the viewpoint of separation performance, the hollow fiber type is preferable.

Generally, when a polymer that suppresses the adherence of platelets or proteins is introduced on the surface of the membrane, the eluate from the hollow fiber membrane module increases. Thus, when the amount of the polymer eluted from the hollow fiber membrane module is large, the eluate enters blood and may cause side effects and complications during use in blood purification application such as dialysis. However, when the polymer A comprising the hydrophilic unit and hydrophobic unit and having an alkyl group having 2 to 20 carbon atoms at a side-chain terminal is introduced, the eluate does not increase significantly. It is thought that elution is suppressed due to the hydrophobic interaction between the alkyl group at a side-chain terminal and the hydrophobic polymer of the hollow fiber membrane. The amount of the polymer eluted is preferably 1.0 mg/$m^2$ or less, more preferably 0.8 mg/$m^2$ or less, further preferably 0.7 mg/$m^2$ or less. Most preferably, the amount of the polymer eluted is 0.0 mg/$m^2$.

The amount of eluate contained in the water circulated inside the hollow fiber membrane module for 4 hours is taken as the amount of the eluate of the hollow fiber membrane module. This measurement reveals the amount of the polymer eluted from the hollow fiber membrane during use of the hollow fiber membrane module. The water circulated for 4 hours refers to the water obtained as follows: ultrapure water is passed through the flow path of the inner surface of the hollow fiber membrane of the hollow fiber membrane module at 100 ml/min for 5 minutes, and then is passed from the inner surface to the outer surface of the hollow fiber membrane at 100 ml/min for 5 minutes in the same way, and 4 liters of ultrapure water heated to 37°C is passed through the inner surface side of the hollow fiber membrane at 200 ml/min and is circulated for 4 hours, thereby the water circulated for 4 hours is obtained. The eluate eluted in water can be measured by gel filtration chromatography or the like using the liquid obtained by concentrating the water circulated for 4 hours 100 times as a measurement sample. The amount of the eluate (mg/$m^2$) is calculated by the following formula. The amount of the eluate is rounded off to the first decimal place.

Amount of eluate (mg/$m^2$) = amount of polymer eluted in 4 L of water (mg)/total of inner surface area of hollow fiber membrane ($m^2$)

The main raw material of the separation membrane is preferably a polysulfone-based polymer. The "polysulfone-based polymer" refers to a polymer having an aromatic ring, a sulfonyl group, and an ether group in the main chain, and examples of the polysulfone-based polymer include polysulfone, polyether sulfone, and polyaryl ether sulfone. The "main raw material" refers to a raw material contained in an amount of 90% by weight or more in the entire separation membrane.

As the main raw material of the separation membrane, for example, a polysulfone-based polymer represented by the chemical formulas of the following formula (1) and/or (2) is preferably used, but it is not limited thereto. In the formulas, n is an integer of 1 or more, and is preferably 30 to 100, more preferably 50 to 80. When n has a distribution, the average is taken as n.

[Chemical formula 1]

(1)

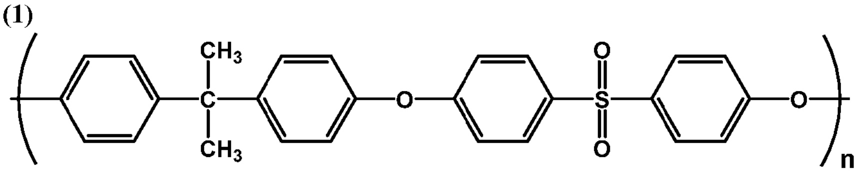

(2)

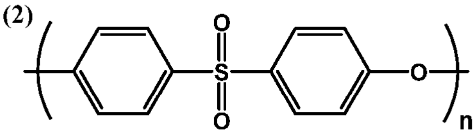

where n represents an integer of 1 or more.

The polysulfone-based polymer that can be used in the separation membrane module is preferably a polymer composed of only the repeating units represented by the above formula (1) and/or (2). However, the polysulfone-based polymer may be a copolymer or a denatured copolymer obtained by copolymerization with a monomer other than the

monomer derived from the repeating units represented by the above formula (1) and/or (2), as long as the effect of the present invention is not impaired. The copolymerization rate of the monomer other than the monomer derived from the repeating units represented by the above formulas (1) and/or (2) in the copolymer obtained by copolymerization with the monomer other than the monomer derived from the repeating units represented by the above formulas (1) and/or (2) is preferably 10% by weight or less in the entire polysulfone-based polymer.

Examples of the polysulfone-based polymer that can be used in the separation membrane module include polysulfone polymers such as Udel Polysulfone P-1700, P-3500 (manufactured by SOLVAY), Ultrason (registered trademark) S3010 or S6010 (manufactured by BASF), VICTREX (manufactured by Sumitomo Chemical Co., Ltd.), Radel (registered trademark) A (manufactured by SOLVAY), or Ultrason (registered trademark) E (manufactured by BASF).

A schematic diagram of a section horizontal to the longitudinal direction of a hollow fiber membrane module (17) which is one of the forms of the separation membrane module is shown in Fig. 1. The hollow fiber membrane module has a structure in which a plurality of hollow fiber membranes (12) cut into a predetermined length are bundled in a tubular case (11) and both terminals of the hollow fiber membranes are each fixed by a potting agent (16). Both terminals of the hollow fiber membrane (12) are open. Headers (13A and 13B) are attached to both terminals of the hollow fiber membrane module, and the headers have a hollow fiber membrane blood side inlet (14A) and a hollow fiber membrane blood side outlet (14B). The tubular case (11) has a hollow fiber membrane dialysate side inlet (15A) and a hollow fiber membrane dialysate side outlet (15B).

As a method of producing the separation membrane module, there are various methods depending on its use. As one aspect, the method can be divided into a production step of the separation membrane and a step of incorporating the separation membrane into a module. In the production of the separation membrane module, the treatment by radiation may be performed before the step of incorporating the separation membrane into the module, or may be performed after the step of incorporating the separation membrane into the module. In particular, when the separation membrane module is for medical use, it is preferable from the viewpoint that sterilization can be also performed at the same time that a treatment by $\gamma$-radiation be performed as the treatment by radiation after the step of incorporating the separation membrane into the module.

An example of the method of producing the hollow fiber membrane module will be shown.

As the method of producing the hollow fiber membrane, for example, the following method is included. That is, a dope solution (the concentration is preferably 10 to 30% by weight, more preferably 15 to 25% by weight) obtained by dissolving polysulfone and polyvinylpyrrolidone (the weight ratio is preferably 20: 1 to 1: 5, more preferably 5: 1 to 1: 1) in a mixed solution of a good solvent (N,N-dimethylacetamide, dimethylsulfoxide, N,N-dimethylformamide, N-methylpyrrolidone, dioxane or the like is preferable) and a poor solvent (water, ethanol, methanol, glycerin or the like is preferable) of polysulfone is discharged from a double annular mouthpiece with bore liquid being flowed inside, run on a dry part, and then guided to a coagulation bath. At this time, because the membrane is affected by the humidity of the dry part, it is also possible to accelerate the phase separation behavior in the vicinity of the outer surface by water supply from the outer surface of the membrane during running on the dry part, increase the hole diameter, and consequently reduce permeation/diffusion resistance during dialysis. However, when the relative humidity is too high, coagulation of the dope solution on the outer surface becomes dominant, the hole diameter rather becomes smaller, and as a result, permeation/diffusion resistance during dialysis tend to increase. Therefore, the relative humidity is preferably 60 to 90%. As the bore liquid, one having a composition based on the solvent used for the dope solution is preferably used from the viewpoint of process suitability. For the concentration of the bore liquid, for example, when N,N-dimethylacetamide is used, a solution of 45 to 80% by weight is preferably used, and a solution of 60 to 75% by weight is more preferably used.

The good solvent is a solvent in the membrane forming dope solution in which the polysulfone-based polymer can be dissolved, and is preferably a solution in which 10% by weight or more of the polysulfone-based polymer can be dissolved. Without particular limitation, N,N-dimethylacetamide and N-methylpyrrolidone are preferably used from the viewpoint of solubility. Meanwhile, the poor solvent is a solvent in the membrane forming dope solution in which the polysulfone-based polymer cannot be dissolved, and is preferably a solution in which 0.1% by weight or more of the polysulfone-based polymer cannot be dissolved. Without particular limitation, water is preferably used.

Examples of the method of incorporating the hollow fiber membrane into the module include, without particular limitation, the following method. First, a hollow fiber membrane is cut to a required length, and a necessary number of the hollow fiber membrane is bundled and then placed in a tubular case. Then, temporary caps are placed at both terminals, and a potting agent is placed at both terminals of the hollow fiber membrane. At this time, a method of placing the potting agent while rotating the module with a centrifuge is preferable because the potting agent is uniformly filled. After the potting agent solidified, both terminals are cut so that both terminals of the hollow fiber membrane are opened, thereby the hollow fiber membrane module is obtained.

When the polymer A has an ester group, an infrared absorption peak derived from the ester group C=O appears in the range of 1711 to 1751 $cm^{-1}$. When the hydrophobic polymer has an aromatic group like a polysulfone-based polymer, an infrared absorption peak derived from the aromatic group C=C appears in the range of 1549 to 1620 $cm^{-1}$.

When the surface fixation amount of the polymer A on the surface of the separation membrane is quantified by ATR-IR, the ratio $(A_{C=O})/(A_{C=C})$ of the infrared absorption peak area $(A_{C=O})$ derived from the ester group C=O of 1711 to 1751 $cm^{-1}$ to the infrared absorption peak area $(A_{C=C})$ derived from the aromatic group C=C of 1549 to 1620 $cm^{-1}$ is measured at any 3 points on the surface of the functional layer of the same medical material, and the average is taken as the surface fixation amount of the polymer A.

To suppress the deterioration over time of the separation membrane, the surface fixation amount of the polymer A, that is, the average of $(A_{C=O})/(A_{C=C})$ is preferably 0.01 or more, more preferably 0.02 or more, further preferably 0.03 or more. The upper limit of the surface fixation amount of the polymer A is not particularly limited. However, when the surface fixation amount of the polymer A is too large, the eluate may increase. Thus, the surface fixation amount of the polymer A is preferably 1.0 or less, more preferably 0.9 or less, further preferably 0.8 or less. Any preferred lower limit can be combined with any preferred upper limit.

The present invention provides a separation membrane module for blood purification.

The "for blood purification" means that the separation membrane module is used for the purpose of removing waste products and harmful substances in blood.

The "separation membrane module for blood purification" refers to a separation membrane module intended to remove waste products and harmful substances in blood by circulating blood outside the body. Examples of the separation membrane module include an artificial kidney module, an artificial liver module, an artificial lung module, and a plasma separation membrane module. In particular, the separation membrane module of the present invention is excellent in removing water and waste products such as urea and creatinine in blood, and is preferably used as the artificial kidney module or the plasma separation membrane module.

The separation membrane module for blood purification is used for about 4 hours as the artificial kidney module used for treatment of chronic renal failure, and is used for one to several days as a continuous blood purifiers used for treatment of acute renal failure, and thus it is used in contact with blood for a long time. Therefore, due to the adherence of platelets or proteins, the fractionation performance and the water permeation performance deteriorate. Furthermore, because, in the artificial kidney module and the continuous blood purifiers, blood is filtered from the inside to the outside of the hollow fiber membrane for the purpose of removing waste products and harmful substances in the blood, the adherence of platelets or proteins is particularly likely to occur.

The separation membrane module of the present invention has little deterioration in performance even when in contact with biological components such as blood for a long time, and thus is preferably used for the slow continuous type blood filter that is used for a long time.

EXAMPLES

Hereinafter, the present invention will be described by way of examples, but the present invention is not limited by these examples.

<Evaluation method>

(1) NMR measurement

A polymer (2 mg) was dissolved in 2 ml of chloroform-D, 99.7% (manufactured by Wako Pure Chemical Industries, Ltd., containing 0.05 V/V% TMS), and the solution was placed in an NMR sample tube, and subjected to NMR measurement (manufactured by JEOL Ltd., superconducting FTNMR EX- 270). The temperature was room temperature, and the integration number was 32 times.

(2) Number average molecular weight

A 0.1 N $LiNO_3$ solution of water/methanol = 50/50 (volume ratio) was prepared and used as a GPC developing solution. A polymer (2 mg) was dissolved in 2 ml of this solution. This solution (100 $\mu$L) was injected into Prominence GPC system manufactured by Shimadzu Corporation and subjected to measurement. The instrument configuration was as follows.

Pump: LC-20AD
Autosampler: SIL-20AHT
Column oven: CTO-20A
Column: GMPWXL (inner diameter: 7.8 mm $\times$ 30 cm, particle diameter: 13 $\mu$m) manufactured by TOSOH CORPORATION.

The flow rate was 0.5 ml/min, the measurement time was 30 minutes, and the column temperature was 40°C. Detection was performed with a differential refractometer RID-10A (manufactured by Shimadzu Corporation), and the number average molecular weight was calculated from the peak derived from the polymer that appeared around the elution time of 15 minutes. The number average molecular weight was rounded off to the nearest thousand. A calibration curve was produced using a polyethylene oxide standard sample (0.1 kD to 1258 kD) manufactured by Agilent Technologies, Inc.

(3) Retention rate of albumin sieving coefficient at 60 minutes after circulation start relative to albumin sieving coefficient at 10 minutes after circulation start

The retention rate of an albumin sieving coefficient at 60 minutes after circulation start relative to an albumin sieving coefficient at 10 minutes after circulation start was measured as follows. First, a hollow fiber membrane module (21) and a blood circuit were connected as shown in Fig. 2. Bovine blood was prepared by collecting raw blood from beef cattle (Japanese, about 30 months after birth) and adding 15% by volume of citric acid (ACD-A solution, manufactured by TERUMO CORPORATION). Bovine blood to which 50 U/ml of heparin was added and which has a hematocrit of 30% by volume and a total protein concentration of 6 to 7 g/dl was prepared, and placed in a circulation beaker (24). The circulation beaker (24) containing the bovine blood was kept at 37°C in a warm water bath (29) equipped with a heater (28).

The inlet of a Bi circuit (25), the outlet of a Bo circuit (26), and the outlet of a F circuit (27) were placed in the circulation beaker (24) containing 2 L of the bovine blood prepared above, and a Bi pump (22) was started at a circulation flow rate of 100 ml/min.

Subsequently, a F pump (23) was started at a filtration flow rate of 10 ml/(min·$m^2$), and samples were each collected from the inlet of the Bi circuit (25), the outlet of the Bo circuit (26), and the outlet of the F circuit (27) over time. The blood sampled at the inlet of the Bi circuit (25) and the outlet of the Bo circuit (26) was centrifuged at 3000 rpm for 10 minutes, and then the supernatant plasma was collected and subjected to albumin concentration measurement. The filtration flow rate of 10 ml/(min·$m^2$) refers to filtration at 10 ml/min per 1.0 $m^2$ of the separation membrane. When the separation membrane module has a membrane area of 1.3 $m^2$, filtration is performed at 13 ml/min.

The albumin concentration at each elapsed time from the start of the F pump (23) was measured, and the albumin sieving coefficient (ScAlbs) at each elapsed time was calculated by the following formula.

$$\text{ScAlb}\ (\%) = 2 \times \text{CF}/(\text{CBi} + \text{CBo}) \times 100$$

In the above formula, CF refers to the albumin concentration (g/ml) at the outlet of the F circuit (27), CBo refers to the albumin concentration (g/ml) at the outlet of the Bo circuit (26), and CBi refers to the albumin concentration (g/ml) at the inlet of the Bi circuit (25). The albumin concentration was measured by the BCG method using an albumin coloring solution of A/G B-Test Wako (manufactured by Wako Pure Chemical Industries, Ltd.). The calibration curve was prepared using the accompanying standard serum diluted with distilled water.

The retention rate of an albumin sieving coefficient (ScAlb60) at 60 minutes after circulation start relative to an albumin sieving coefficient (ScAlb10) at 10 minutes after circulation start was calculated by the following formula. The retention rate was rounded off to the nearest whole number.

$$\text{Retention rate}\ (\%) = \text{ScAlb60}/\text{ScAlb10} \times 100$$

(4) Retention rate of albumin sieving coefficient at 1440 minutes after circulation start relative to albumin sieving coefficient at 10 minutes after circulation start

The measurement was performed in the same manner as in the measurement of the retention rate of an albumin sieving coefficient at 60 minutes after circulation start relative to an albumin sieving coefficient at 10 minutes after circulation start except that the bovine plasma which was prepared to have a total protein concentration of 6 to 7 g/dl and to which 50 U/ml of heparin was added was used instead of bovine blood. The retention rate of an albumin sieving coefficient (ScAlb1440') at 1440 minutes after circulation start relative to an albumin sieving coefficient (ScAlb10') at 10 minutes after circulation start was calculated by the following formula. The retention rate was rounded off to the nearest whole number.

$$\text{Retention rate}\ (\%) = \text{ScAlb1440}'/\text{ScAlb10}' \times 100$$

(5) Test method of adherence of platelets

A double-faced tape was attached to a circular plate made of polystyrene having 18 mmφ, and a hollow fiber

membrane was fixed thereto. The attached hollow fiber membrane was shaved and cut into a semi-cylindrical shape with a single edged knife to expose the inner surface of the hollow fiber membrane. When the inner surface of the hollow fiber membrane has dirt, scratches, folds and the like, platelets may adhere there, which may result in incorrect evaluation. Thus, the hollow fiber membrane without dirt, scratches or folds was used. The circular plate was placed on Falcon (registered trademark) tube (18 mmφ, No. 2051) cut into a tubular shape with the side on which the hollow fiber membrane was attached facing to the inside of the tube, and the gap was filled with parafilm. The inside of the tube was washed with saline, and then was filled with saline. Healthy human venous blood was collected and then immediately 50 U/ml of heparin was added thereto. The saline in the tube was discarded, and then 1.0 ml of the blood was placed in the tube within 10 minutes after the blood collection and shaken at 37°C for 1 hour. Thereafter, the hollow fiber membrane was washed with 10 ml of saline, blood components were fixed with 2.5% glutaraldehyde saline, and the hollow fiber membrane was washed with 20 ml of distilled water. The washed hollow fiber membrane was dried under reduced pressure at 20°C and 0.5 Torr for 10 hours. The hollow fiber membrane was attached to a stage of a scanning electron microscope with double-faced tape. Thereafter, a thin film of Pt-Pd was formed on the surface of the hollow fiber membrane by sputtering to prepare a sample. The inner surface of the hollow fiber membrane was observed with a field emission type scanning electron microscope (S800, manufactured by Hitachi, Ltd.) at a magnification of 1500 times, and the number of platelets adhered within one field of view ($4.3 \times 10^3$ $\mu m^2$) was counted. When 50 or more platelets were adhered, the number was regarded as 50 assuming that the hollow fiber membrane has no effect of suppressing the adherence of platelets. The average of the numbers of an adhered platelet in 20 different fields of view around the center in the longitudinal direction of the hollow fiber membrane was taken as the number of an adhered human platelet (number/$4.3 \times 10^3$ $\mu m^2$). When an electron microscope having a different area of a field of view is used, the area can be appropriately converted to obtain the number of an adhered platelet (number/$4.3 \times 10^3$ $\mu m^2$). When the membrane is not a hollow fiber membrane, the inner surface of the separation membrane is appropriately exposed and brought into contact with the blood, and the number of an adhered platelet can be counted.

In the platelet adherence test, in order to confirm whether the test is performed properly, a positive control and a negative control are considered as levels at each experiment. The positive control is a known sample known to have a high number of an adhered platelet. The negative control is a known sample known to have a small adherence number of a platelet. As the positive control, a hollow fiber membrane of "Filtryzer" BG (manufactured by TORAY INDUSTRIES, INC.) was used, and as the negative control, a hollow fiber membrane of "Toraylight" CX (manufactured by TORAY INDUSTRIES, INC.) was used. Under the above experimental conditions, when the number of an adhered platelet in the positive control was 40 (number/$4.3 \times 10^3$ $\mu m^2$) or more and the number of an adhered platelet in the negative control was 20 (number/$4.3 \times 10^3$ $\mu m^2$) or less, the measured values were used. When the numbers of an adhered platelet in the controls were not within the above range, the test was redone because the blood was possibly not fresh or was excessively activated.

(6) Measurement of thickness of swelling layer

The hollow fiber membrane was shaved and cut into a semi-cylindrical shape with a single edged knife to measure the inner surface of the hollow fiber membrane. The hollow fiber membrane was attached to a stage and then the membrane surface was wet with water. In this state, the force curve measurement was performed in the AFM contact mode (Fig. 3). When the cantilever approaches the sample and the swelling layer is present on the surface, a curved portion (32) is observed between a linear region (31) that is produced before the cantilever comes into contact with the surface and a rear linear region (33) that is produced after the cantilever comes into contact with the surface. The distance from the intersection point between the extension lines of the two lines to the start of the curved portion was defined as a thickness of the swelling layer (34). The measurement was performed at 5 arbitrarily selected points on a plurality of arbitrarily selected hollow fiber membranes, and the average was taken as the thickness. When the separation membrane is not a hollow fiber membrane, the measurement is performed at 5 arbitrarily selected points on the inner surface of the separation membrane, and the average is taken as the thickness. The average was rounded off to the nearest whole number. The measuring instrument and measuring conditions were as follows.

Scanning probe microscopy SPM 9500-J3 (SHIMADZU, Kyoto, Japan)

Observation mode: contact mode
Probe: NP-S (120 mm, wide) (Nihon VEECO KK, Tokyo, Japan)
Scan range: 5 $\mu m \times 5$ $\mu m$, scan speed: 1 Hz

(7) Water permeability measurement

For the separation membrane module that is a hollow fiber membrane module, the blood side inlet/outlet of the hollow fiber membrane module were connected to the circuit, and the hollow fiber membrane module was washed with water at 200 ml/min for 5 minutes or more. Then, water (37°C) was flowed at 200 ml/min, the outflow at the blood side outlet was adjusted, and the filtration amount V per minute that is the amount flowing out to the dialysate inlet side per minute and the average pressure P at the blood side inlet/outlet were measured. Ultrafiltration rate UFR was calculated by the following formula. The outflow from the blood side outlet was changed, measurement was performed at three points, and the average of UFRs was taken as the water permeability of the hollow fiber membrane module. When the separation membrane module is not a hollow fiber membrane module, the separation membrane module is washed with water and the water is passed through the separation membrane module under the same conditions as above, UFRs are calculated, and the average of UFRs at three points with the outflow from the blood side outlet changed can be taken as the water permeability of the separation membrane module.

$$\text{UFR (ml/hr/mmHg/m}^2\text{)} = V \times 60/P/A$$

V: filtration amount (ml/min), P: pressure (mmHg), A: membrane area ($m^2$)

(8) Eluate test

For the separation membrane module that is a hollow fiber membrane module, ultrapure water was passed through the flow path on the inner surface side of the hollow fiber membrane at 100 ml/min for 5 minutes, and then was passed from the inner surface to the outer surface side of the hollow fiber membrane at 100 ml/min for 5 minutes in the same way to wash the hollow fiber membrane module. Thereafter, 4 L of ultrapure water heated to 37°C was passed and circulated through the inner surface side of the hollow fiber membrane at 200 ml/min for 4 hours with being circulated. Water after the circulation of 4 hours was collected to obtain a sample solution. Because the obtained sample solution was dilute, the solution was lyophilized, concentrated 100-fold, and then subjected to gel filtration chromatography measurement. Gel filtration chromatography was performed under the following conditions. First, measurement was performed by gel filtration chromatography using several kinds of aqueous solutions in which polyvinylpyrrolidone (K90, manufactured by ISP Corporation) was dissolved at different concentrations of 10 to 1000 ppm as standard samples. A calibration curve for the relationship between the peak area of polyvinylpyrrolidone and the prepared concentrations of the standard samples was produced. Next, the concentration of the eluate in the sample solution was calculated from the peak area derived from the eluate obtained by measuring the sample solution and the above calibration curve. When the separation membrane module is not a hollow fiber membrane module, the flow path on the inner surface side of the separation membrane may be washed and water may be passed there under the same conditions as above to calculate the concentration of the eluate in the sample solution.

Subsequently, the amount of the eluted polymer contained in 4 L of ultrapure water after the circulation of 4 hours was calculated by the following formula. The calculation was rounded off to the first decimal place.

Amount of eluted polymer in 4 L of water (mg) = polymer concentration in measurement sample (ppm) × 4 (kg)/100

Amount of eluate (mg/$m^2$) = amount of polymer eluted in 4 L of water (mg)/total of inner surface area of hollow fiber membrane ($m^2$)

Column: TSK gel GMPWXL (manufactured by TOSOH CORPORATION, inner diameter: 7.8 mm × 30 cm, particle diameter: 7 μm)
Solvent: 0.1 mol/L lithium nitrate, water/methanol: 50 vol/50 vol
Flow rate: 0.5 ml/min
Column temperature: 40°C
Detector: differential refractometer RI-8010

(manufactured by TOSOH CORPORATION)

(9) ATR-IR measurement

For the separation membrane that is a hollow fiber membrane, the hollow fiber membrane was shaved and cut into a semi-cylindrical shape with a single edged knife, rinsed with ultrapure water, and then dried at room temperature and 0.5 Torr for 10 hours to obtain a sample for surface measurement. The inner surface of this dried hollow fiber membrane

was subjected to measurement by micro ATR method using IRT-3000 manufactured by JASCO Corporation. In the measurement, the field of view (the aperture) was 100 μm × 100 μm, the measurement range was 3 μm × 3 μm, and the integration number was 30 times. A base line was drawn at the wavelength of 1549 to 1620 $cm^{-1}$ in the obtained spectrum, and the part surrounded by the base line and the positive part of the spectrum was defined as the peak area derived from the polysulfone-derived aromatic group C=C ($A_{C=C}$). In the same way, a base line was drawn at 1711 to 1751 $cm^{-1}$, and the part surrounded by the base line and the positive part of the spectrum was defined as the peak area derived from the ester group ($A_{C=O}$). Depending on the type of the vinyl carboxylate ester unit and the type of the polysulfone-based polymer, the peak may be shifted by about ± 10 $cm^{-1}$. In such a case, the base line is redrawn as appropriate. The measurement by the above procedure was performed at three different places in the same hollow fiber membrane, and the average of ($A_{C=O}$)/($A_{C=C}$) was calculated and rounded off to the second decimal place. When the separation membrane is not a hollow fiber membrane, the measurement can be performed at 3 arbitrarily selected points on the inner surface of the separation membrane, and the average of ($A_{C=O}$)/($A_{C=C}$) can be used.

<Method of Producing Hollow Fiber Membrane Module>

To 72 parts by weight of N,N-dimethylacetamide and 1 part by weight of water, 18 parts by weight of polysulfone (Udel P-3500, manufactured by Teijin Amoco Co., Ltd.) and 9 parts by weight of polyvinylpyrrolidone (K30, manufactured by BASF) were added, and the mixture was heated and dissolved at 90°C for 14 hours. The membrane forming dope solution was discharged from an orifice-type double cylindrical mouthpiece having an outer diameter of 0.3 mm and an inner diameter of 0.2 mm, a solution containing 57.5 parts by weight of N,N-dimethylacetamide and 42.5 parts by weight of water was discharged as a bore liquid, and both solutions were passed through a dry length of 350 mm, and then led to a coagulation bath of 100% water to obtain a hollow fiber membrane. The inner diameter of the obtained hollow fiber membrane was 200 μm, and the membrane thickness was 40 μm. The hollow fiber membrane was filled in a case (the inner diameter of the case body: 36 mm) so that the total of the inner surface area was 1.0 $m^2$, the number of hollow fibers was about 8200, and the effective length was 195 mm. Then potting was performed, and both terminals were opened to obtain a hollow fiber membrane module.

The following examples 1-7 and 9 are reference examples.

(Example 1)

A vinylpyrrolidone/vinyl pentanoate random copolymer was produced by the following method. A vinylpyrrolidone monomer (14.5 g) (manufactured by Wako Pure Chemical Industries, Ltd.), a vinyl pentanoate monomer (22.5 g) (manufactured by Sigma-Aldrich Co. LLC.), isopropanol (56 g) (manufactured by Wako Pure Chemical Industries, Ltd.) as a polymerization solvent, and azobisdimethylbutyronitrile (0.31 g) as a polymerization initiator were mixed and stirred at 70°C for 6 hours under a nitrogen atmosphere. The reaction liquid was cooled to room temperature, and concentrated, then the concentrated residue was poured into hexane. The deposited white precipitate was collected and dried under reduced pressure at 60°C for 12 hours to obtain a vinylpyrrolidone/vinyl pentanoate random copolymer. The measurement result of $^1$H-NMR showed that the mole fraction of the vinylpyrrolidone unit in the whole copolymer was 60%. The measurement result of GPC showed that the number average molecular weight of the copolymer was 3,900.

A hollow fiber membrane module in which the produced vinylpyrrolidone/vinyl pentanoate random copolymer is introduced on the surface of the polysulfone hollow fiber was produced by the following method. A 1.0% by weight ethanol aqueous solution in which 300 ppm of the copolymer was dissolved was passed from a blood side inlet (14A) to the dialysate side inlet (15A) of the hollow fiber membrane module (Fig. 1) produced by the method of producing the hollow fiber membrane module. Further, a 0.1% by weight ethanol aqueous solution was passed from the blood side inlet (14A) to the dialysate side inlet (15A) and from the blood side inlet (15A) to the blood side outlet (14B) of the hollow fiber membrane module, and then 25 kGy of y rays was irradiated there to produce a hollow fiber membrane module.

The measurement result of ATR-IR on the inner surface of the hollow fiber membrane of the produced hollow fiber membrane module showed that peaks were present in the ranges of 1711 to 1751 $cm^{-1}$ and 1549 to 1620 $cm^{-1}$. The measurement result of ATR-IR showed that the copolymer fixation amount on the inner surface of the hollow fiber membrane (the average of the ratio $A_{C=O}/A_{C=C}$ of the peak area $A_{C=O}$ in the range of 1711 to 1751 $cm^{-1}$ to the peak area $A_{C=C}$ in the range of 1549 to 1620 $cm^{-1}$) was 0.07. The force curve measurement of AFM showed that the thickness of the swelling layer on the surface of the separation membrane was 16 nm. The retention rate of an albumin sieving coefficient at 60 minutes after circulation start relative to an albumin sieving coefficient at 10 minutes after circulation start for the bovine blood in the produced hollow fiber membrane module was 90%. The number of an adhered human platelet was 1/4.3 × $10^3$ μ$m^2$, the water permeability was 330 ml/hr/mmHg/$m^2$, and the amount of the eluate was 0.5 mg/$m^2$.

(Example 2)

A vinylpyrrolidone/vinyl propanoate random copolymer was prepared by the following method. A vinylpyrrolidone monomer (19.5 g), a vinyl propanoate monomer (17.5 g), t-amyl alcohol (56 g) as a polymerization solvent, and 2,2'-azobis (2,4-dimethylvaleronitrile) (0.175 g) as a polymerization initiator were mixed and stirred at 70°C for 5 hours under a nitrogen atmosphere. The reaction liquid was cooled to room temperature to stop the reaction, concentrated, and then poured into hexane. The deposited white precipitate was collected and dried under reduced pressure to obtain a vinylpyrrolidone/vinyl propanoate random copolymer.
The measurement result of $^1$H-NMR showed that the mole fraction of the vinylpyrrolidone unit in the whole copolymer was 60%. The measurement result of GPC showed that the number average molecular weight of the copolymer was 16,500.
A hollow fiber membrane module was produced in the same manner as in Example 1 using the obtained copolymer.
The measurement result of ATR-IR showed that peaks were present in the ranges of 1711 to 1751 $cm^{-1}$ and 1549 to 1620 $cm^{-1}$. The measurement result of ATR-IR showed that the copolymer fixation amount on the inner surface of the hollow fiber membrane (the average of the ratio $A_{C=O}/A_{C=C}$ of the peak area $A_{C=O}$ in the range of 1711 to 1751 $cm^{-1}$ to the peak area $A_{C=C}$ in the range of 1549 to 1620 $cm^{-1}$) was 0.06. The force curve measurement of AFM showed that the thickness of the swelling layer on the surface of the separation membrane was 11 nm. The retention rate of an albumin sieving coefficient at 60 minutes after circulation start relative to an albumin sieving coefficient at 10 minutes after circulation start for the bovine blood in the produced hollow fiber membrane module was 98%. The number of an adhered human platelet was $0/4.3 \times 10^3\ \mu m^2$, the water permeability was 410 ml/hr/mmHg/$m^2$, and the amount of the eluate was 0.6 mg/$m^2$. Further, the retention rate of an albumin sieving coefficient at 1440 minutes after circulation start relative to an albumin sieving coefficient at 10 minutes after circulation start for the bovine plasma in the hollow fiber membrane module was 97%.

(Example 3)

A hollow fiber membrane module was produced in the same manner as in Example 2 except that the concentration of the core liquid at the time of forming the hollow fiber membrane was 54 parts by weight of N,N-dimethylacetamide and 46 parts by weight of water, and a vinylpyrrolidone/vinyl propanoate random copolymer (number average molecular weight: 7,600, mole fraction of the vinylpyrrolidone unit in the whole copolymer: 60%) was used instead of the vinylpyrrolidone/vinyl propanoate random copolymer (number average molecular weight: 16,500, vinylpyrrolidone unit: 60%).
The measurement result of ATR-IR showed that peaks were present in the ranges of 1711 to 1751 $cm^{-1}$ and 1549 to 1620 $cm^{-1}$. The measurement result of ATR-IR showed that the copolymer fixation amount on the inner surface of the hollow fiber membrane (the average of the ratio $A_{C=O}/A_{C=C}$ of the peak area $A_{C=O}$ in the range of 1711 to 1751 $cm^{-1}$ to the peak area $A_{C=C}$ in the range of 1549 to 1620 $cm^{-1}$) was 0.07. The force curve measurement of AFM showed that the thickness of the swelling layer on the surface of the separation membrane was 10 nm. The retention rate of an albumin sieving coefficient at 60 minutes after circulation start relative to an albumin sieving coefficient at 10 minutes after circulation start for the bovine blood in the produced hollow fiber membrane module was 93%. The number of an adhered human platelet was $1/4.3 \times 10^3\ \mu m^2$, the water permeability was 310 ml/hr/mmHg/$m^2$, and the amount of the eluate was 0.5 mg/$m^2$. Further, the retention rate of an albumin sieving coefficient at 1440 minutes after circulation start relative to an albumin sieving coefficient at 10 minutes after circulation start for the bovine plasma in the hollow fiber membrane module was 89%.

(Example 4)

A hollow fiber membrane module was produced in the same manner as in Example 2 except that a vinylpyrrolidone/vinyl propanoate random copolymer (number average molecular weight: 12,500, mole fraction of the vinylpyrrolidone unit in the whole copolymer: 50%) was used instead of the vinylpyrrolidone/vinyl propanoate random copolymer (number average molecular weight: 16,500, mole fraction of the vinylpyrrolidone unit in the whole copolymer: 60%).
The measurement result of ATR-IR showed that peaks were present in the ranges of 1711 to 1751 $cm^{-1}$ and 1549 to 1620 $cm^{-1}$. The measurement result of ATR-IR showed that the copolymer fixation amount on the inner surface of the hollow fiber membrane (the average of the ratio $A_{C=O}/A_{C=C}$ of the peak area $A_{C=O}$ in the range of 1711 to 1751 $cm^{-1}$ to the peak area $A_{C=C}$ in the range of 1549 to 1620 $cm^{-1}$) was 0.05. The force curve measurement of AFM showed that the thickness of the swelling layer on the surface of the separation membrane was 13 nm. The retention rate of an albumin sieving coefficient at 60 minutes after circulation start relative to an albumin sieving coefficient at 10 minutes after circulation start for the bovine blood in the produced hollow fiber membrane module was 90%. The number of an adhered

human platelet was 0/4.3 $\times$ $10^3$ $\mu m^2$, the water permeability was 400 ml/hr/mmHg/$m^2$, and the amount of the eluate was 0.5 mg/$m^2$.

(Example 5)

A hollow fiber membrane module was produced in the same manner as in Example 2 except that a vinylpyrrolidone/vinyl propanoate random copolymer (number average molecular weight: 12,600, mole fraction of the vinylpyrrolidone unit in the whole copolymer: 70%) was used instead of the vinylpyrrolidone/vinyl propanoate random copolymer (number average molecular weight: 16,500, mole fraction of the vinylpyrrolidone unit in the whole copolymer: 60%).

The measurement result of ATR-IR showed that peaks were present in the ranges of 1711 to 1751 $cm^{-1}$ and 1549 to 1620 $cm^{-1}$. The measurement result of ATR-IR showed that the copolymer fixation amount on the inner surface of the hollow fiber membrane (the average of the ratio $A_{C=O}/A_{C=C}$ of the peak area $A_{C=O}$ in the range of 1711 to 1751 $cm^{-1}$ to the peak area $A_{C=C}$ in the range of 1549 to 1620 $cm^{-1}$) was 0.04. The force curve measurement of AFM showed that the thickness of the swelling layer on the surface of the separation membrane was 15 nm. The retention rate of an albumin sieving coefficient at 60 minutes after circulation start relative to an albumin sieving coefficient at 10 minutes after circulation start for the bovine blood in the produced hollow fiber membrane module was 93%. The number of an adhered human platelet was 1/4.3 $\times$ $10^3$ $\mu m^2$, the water permeability was 370 ml/hr/mmHg/$m^2$, and the amount of the eluate was 0.7 mg/$m^2$.

(Example 6)

A hollow fiber membrane module was produced in the same manner as in Example 2 except that an N-vinylacetamide/vinyl pivalate random copolymer (number average molecular weight: 7,600, mole fraction of the N-vinylacetamide unit in the whole copolymer: 50%) was used instead of the vinylpyrrolidone/vinyl propanoate random copolymer (number average molecular weight: 16,500, mole fraction of the vinylpyrrolidone unit in the whole copolymer: 60%).

The measurement result of ATR-IR showed that peaks were present in the ranges of 1711 to 1751 $cm^{-1}$ and 1549 to 1620 $cm^{-1}$. The measurement result of ATR-IR showed that the copolymer fixation amount on the inner surface of the hollow fiber membrane (the average of the ratio $A_{C=O}/A_{C=C}$ of the peak area $A_{C=O}$ in the range of 1711 to 1751 $cm^{-1}$ to the peak area $A_{C=C}$ in the range of 1549 to 1620 $cm^{-1}$) was 0.06. The force curve measurement of AFM showed that the thickness of the swelling layer on the surface of the separation membrane was 15 nm. The retention rate of an albumin sieving coefficient at 60 minutes after circulation start relative to an albumin sieving coefficient at 10 minutes after circulation start for the bovine blood in the produced hollow fiber membrane module was 96%. The number of an adhered human platelet was 0/4.3 $\times$ $10^3$ $\mu m^2$, the water permeability was 420 ml/hr/mmHg/$m^2$, and the amount of the eluate was 0.3 mg/$m^2$.

(Example 7)

A hollow fiber membrane module was produced in the same manner as in Example 2 except that an N-isopropylacrylamide/ethyl acrylate random copolymer (number average molecular weight: 3,000, mole fraction of the N-isopropylacrylamide unit in the whole copolymer: 50%) was used instead of the vinylpyrrolidone/vinyl propanoate random copolymer (number average molecular weight: 16,500, mole fraction of the vinylpyrrolidone unit in the whole copolymer: 60%).

The measurement result of ATR-IR showed that peaks were present in the ranges of 1711 to 1751 $cm^{-1}$ and 1549 to 1620 $cm^{-1}$. The measurement result of ATR-IR showed that the copolymer fixation amount on the inner surface of the hollow fiber membrane (the average of the ratio $A_{C=O}/A_{C=C}$ of the peak area $A_{C=O}$ in the range of 1711 to 1751 $cm^{-1}$ to the peak area $A_{C=C}$ in the range of 1549 to 1620 $cm^{-1}$) was 0.05. The force curve measurement of AFM showed that the thickness of the swelling layer on the surface of the separation membrane was 10 nm. The retention rate of an albumin sieving coefficient at 60 minutes after circulation start relative to an albumin sieving coefficient at 10 minutes after circulation start for the bovine blood in the produced hollow fiber membrane module was 98%. The number of an adhered human platelet was 0/4.3 $\times$ $10^3$ $\mu m^2$, the water permeability was 360 ml/hr/mmHg/$m^2$, and the amount of the eluate was 0.4 mg/$m^2$.

(Example 8)

A hollow fiber membrane module was produced in the same manner as in Example 2 except that an N-methylacrylamide/propyl methacrylate random copolymer (number average molecular weight: 4,000, mole fraction of the N-methylacrylamide unit in the whole copolymer: 70%) was used instead of the vinylpyrrolidone/vinyl propanoate random

copolymer (number average molecular weight: 16,500, mole fraction of the vinylpyrrolidone unit in the whole copolymer: 60%).

The measurement result of ATR-IR showed that peaks were present in the ranges of 1711 to 1751 $cm^{-1}$ and 1549 to 1620 $cm^{-1}$. The measurement result of ATR-IR showed that the copolymer fixation amount on the inner surface of the hollow fiber membrane (the average of the ratio $A_{C=O}/A_{C=C}$ of the peak area $A_{C=O}$ in the range of 1711 to 1751 $cm^{-1}$ to the peak area $A_{C=C}$ in the range of 1549 to 1620 $cm^{-1}$) was 0.03. The force curve measurement of AFM showed that the thickness of the swelling layer on the surface of the separation membrane was 16 nm. The retention rate of an albumin sieving coefficient at 60 minutes after circulation start relative to an albumin sieving coefficient at 10 minutes after circulation start for the bovine blood in the produced hollow fiber membrane module was 94%. The number of an adhered human platelet was $1/4.3 \times 10^3$ $\mu m^2$, the water permeability was 310 ml/hr/mmHg/$m^2$, and the amount of the eluate was 0.3 mg/$m^2$.

(Example 9)

A hollow fiber membrane module was produced in the same manner as in Example 2 except that an N-vinylacetamide/vinyl octanoate random copolymer (number average molecular weight: 3,000, mole fraction of the N-vinylacetamide unit in the whole copolymer: 60%) was used instead of the vinylpyrrolidone/vinyl propanoate random copolymer (number average molecular weight: 16,500, mole fraction of the vinylpyrrolidone unit in the whole copolymer: 60%).

The measurement result of ATR-IR showed that peaks were present in the ranges of 1711 to 1751 $cm^{-1}$ and 1549 to 1620 $cm^{-1}$. The measurement result of ATR-IR showed that the copolymer fixation amount on the inner surface of the hollow fiber membrane (the average of the ratio $A_{C=O}/A_{C=C}$ of the peak area $A_{C=O}$ in the range of 1711 to 1751 $cm^{-1}$ to the peak area $A_{C=C}$ in the range of 1549 to 1620 $cm^{-1}$) was 0.09. The force curve measurement of AFM showed that the thickness of the swelling layer on the surface of the separation membrane was 12 nm. The retention rate of an albumin sieving coefficient at 60 minutes after circulation start relative to an albumin sieving coefficient at 10 minutes after circulation start for the bovine blood in the produced hollow fiber membrane module was 91%. The number of an adhered human platelet was $2/4.3 \times 10^3$ $\mu m^2$, the water permeability was 320 ml/hr/mmHg/$m^2$, and the amount of the eluate was 0.2 mg/$m^2$.

(Comparative Example 1)

A hollow fiber membrane module was produced in the same manner as in Example 1 except that polyvinylpyrrolidone (K90 manufactured by BASF) was used instead of the vinylpyrrolidone/vinyl pentanoate random copolymer.

The ATR-IR measurement showed that a peak was not present in the range of 1711 to 1751 $cm^{-1}$. The force curve measurement of AFM showed that the thickness of the swelling layer on the surface of the separation membrane was 5 nm. The retention rate of an albumin sieving coefficient at 60 minutes after circulation start relative to an albumin sieving coefficient at 10 minutes after circulation start for the bovine blood in the produced hollow fiber membrane module was 40%. The number of an adhered human platelet was $20/4.3 \times 10^3$ $\mu m^2$, the water permeability was 600 ml/hr/mmHg/$m^2$, and the amount of the eluate was 0.9 mg/$m^2$. Further, the retention rate of an albumin sieving coefficient at 1440 minutes after circulation start relative to an albumin sieving coefficient at 10 minutes after circulation start for the bovine plasma in the hollow fiber membrane module was 28%.

(Comparative Example 2)

A hollow fiber membrane module was produced in the same manner as in Example 1 except that a vinylpyrrolidone/vinyl acetate random copolymer (Kollidon (registered trademark) VA64, manufactured by BASF) was used instead of the vinylpyrrolidone/vinyl pentanoate random copolymer, and the concentration of the copolymer in the aqueous solution was 500 ppm.

The measurement result of ATR-IR showed that peaks were present in the ranges of 1711 to 1751 $cm^{-1}$ and 1549 to 1620 $cm^{-1}$. It was shown that the copolymer fixation amount on the inner surface of the hollow fiber membrane (the average of the ratio $A_{C=O}/A_{C=C}$ of the peak area $A_{C=O}$ in the range of 1711 to 1751 $cm^{-1}$ to the peak area $A_{C=C}$ in the range of 1549 to 1620 $cm^{-1}$) was 0.11. The force curve measurement of AFM showed that the thickness of the swelling layer on the surface of the separation membrane was 17 nm. The retention rate of an albumin sieving coefficient at 60 minutes after circulation start relative to an albumin sieving coefficient at 10 minutes after circulation start for the bovine blood in the produced hollow fiber membrane module was 80%. The number of an adhered human platelet was $2/4.3 \times 10^3$ $\mu m^2$, the water permeability was 300 ml/hr/mmHg/$m^2$, and the amount of the eluate was 2.2 mg/$m^2$.

(Comparative Example 3)

A hollow fiber membrane module was produced in the same manner as in Comparative Example 2 except that the concentration of the copolymer in the aqueous solution was changed to 20 ppm.

The ATR-IR measurement showed that the copolymer fixation amount on the inner surface of the hollow fiber membrane (the average of the ratio $A_{C=O}/A_{C=C}$ of the peak area $A_{C=O}$ in the range of 1711 to 1751 $cm^{-1}$ to the peak area $A_{C=C}$ in the range of 1549 to 1620 $cm^{-1}$) was 0.01. The force curve measurement of AFM showed that the thickness of the swelling layer on the surface of the separation membrane was 7 nm. The retention rate of an albumin sieving coefficient at 60 minutes after circulation start relative to an albumin sieving coefficient at 10 minutes after circulation start for the bovine blood in the produced hollow fiber membrane module was 70%. The number of an adhered human platelet was $6/4.3 \times 10^3$ $\mu m^2$, the water permeability was 540 $ml/hr/mmHg/m^2$, and the amount of the eluate was 0.5 $mg/m^2$. Further, the retention rate of an albumin sieving coefficient at 1440 minutes after circulation start relative to an albumin sieving coefficient at 10 minutes after circulation start for the bovine plasma in the hollow fiber membrane module was 63%.

(Comparative Example 4)

A hollow fiber membrane module was produced in the same manner as in Example 1 except that partially saponified polyvinyl alcohol (PVA417, manufactured by KURARAY CO., LTD.) was used instead of the vinylpyrrolidone/vinyl pentanoate random copolymer.

The ATR-IR measurement showed that the partially saponified polyvinyl alcohol fixation amount on the inner surface of the hollow fiber membrane (the average of the ratio $A_{C=O}/A_{C=C}$ of the peak area $A_{C=O}$ in the range of 1711 to 1751 $cm^{-1}$ to the peak area $A_{C=C}$ in the range of 1549 to 1620 $cm^{-1}$) was 0.03. The force curve measurement of AFM showed that the thickness of the swelling layer on the surface of the separation membrane was 8 nm. The retention rate of an albumin sieving coefficient at 60 minutes after circulation start relative to an albumin sieving coefficient at 10 minutes after circulation start for the bovine blood in the produced hollow fiber membrane module was 77%. The number of an adhered human platelet was $10/4.3 \times 10^3$ $\mu m^2$, the water permeability was 150 $ml/hr/mmHg/m^2$, and the amount of the eluate was 0.5 $mg/m^2$.

(Comparative Example 5)

A hollow fiber membrane module was produced in the same manner as in Example 1 except that polyvinyl acetamide (manufactured by SHOWA DENKO K.K., GE191-053) was used instead of the vinylpyrrolidone/vinyl pentanoate random copolymer.

The ATR-IR measurement showed that a peak was not present in the range of 1711 to 1751 $cm^{-1}$. The force curve measurement of AFM showed that the thickness of the swelling layer on the surface of the separation membrane was 6 nm. The retention rate of an albumin sieving coefficient at 60 minutes after circulation start relative to an albumin sieving coefficient at 10 minutes after circulation start for the bovine blood in the produced hollow fiber membrane module was 45%. The number of an adhered human platelet was $40/4.3 \times 10^3$ $\mu m^2$, the water permeability was 580 $ml/hr/mmHg/m^2$, and the amount of the eluate was 0.7 $mg/m^2$.

The composition of the polymer A used in each example and each comparative example is shown in Table 1, and the results of each example and each comparative example are shown in Table 2.

[Table 1]

| | Polymer A | | | |
|---|---|---|---|---|
| | **Hydrophilic unit** | **Hydrophobic unit** | **Mole fraction of hydrophilic unit (%)** | **Carbon number of alkyl group** |
| **Example 1** | **Vinylpyrrolidone** | **Vinyl pentanoate** | **60** | **4** |
| **Example 2** | **Vinylpyrrolidone** | **Vinyl propanoate** | **60** | **2** |
| **Example 3** | **Vinylpyrrolidone** | **Vinyl propanoate** | **60** | **2** |
| **Example 4** | **Vinylpyrrolidone** | **Vinyl propanoate** | **50** | **2** |
| **Example 5** | **Vinylpyrrolidone** | **Vinyl propanoate** | **70** | **2** |
| **Example 6** | **N-vinylacetamide** | **Vinyl pivalate** | **50** | **4** |
| **Example 7** | **N-isopropylacrylamide** | **Ethyl acrylate** | **50** | **2** |

(continued)

| | Polymer A | | | |
|---|---|---|---|---|
| | Hydrophilic unit | Hydrophobic unit | Mole fraction of hydrophilic unit (%) | Carbon number of alkyl group |
| Example 8 | N-methylacrylamide | Propyl methacrylate | 70 | 3 |
| Example 9 | N-vinylacetamide | Vinyl octanoate | 60 | 7 |
| Comparative Example 1 | Vinylpyrrolidone | - | 100 | o |
| Comparative Example 2 | Vinylpyrrolidone | Vinyl acetate | 60 | 1 |
| Comparative Example 3 | Vinylpyrrolidone | Vinyl acetate | 60 | 1 |
| Comparative Example 4 | Vinyl alcohol | Vinyl acetate | 80 | 1 |
| Comparative Example 5 | N-vinylacetamide | - | 100 | 0 |

In Table 1, the mole fraction of the hydrophilic unit in the whole copolymer is described as "mole fraction of hydrophilic unit", and the number of carbon atoms of the alkyl group at a side-chain terminal of the hydrophobic unit is described as "carbon number of alkyl group".

[Table 2]

| | Hollow fiber membrane module | | | | |
|---|---|---|---|---|---|
| | Retention rate of albumin sieving coefficient (3) | Number of an adhered human platelet (number/ 4.3 $\times$ $10^3$ $gm^2$) | Water permeability ($ml/hr/mmEg/m^2$) | Amount of eluate ($mg/m^2$) | Copolymer fixation amount |
| Example 1 | 90 | 1 | 330 | 0.5 | 0.07 |
| Example 2 | 98 | 0 | 410 | 0.6 | 0.06 |
| Example 3 | 93 | 1 | 310 | 0.5 | 0.07 |
| Example 4 | 90 | 0 | 400 | 0.5 | 0.05 |
| Example 5 | 93 | 1 | 370 | 0.7 | 0.04 |
| Example 6 | 96 | 0 | 420 | 0.3 | 0.06 |
| Example 7 | 98 | 0 | 360 | 0.4 | 0.05 |
| Example 8 | 94 | 1 | 310 | 0.3 | 0.03 |
| Example 9 | 91 | 2 | 320 | 0.2 | 0.09 |
| Comparative Example 1 | 40 | 20 | 600 | 0.9 | - |
| Comparative Example 2 | 80 | 2 | 300 | 2.2 | 0.11 |
| Comparative Example 3 | 70 | 6 | 540 | 0.5 | 0.01 |
| Comparative Example 4 | 77 | 10 | 150 | 0.5 | 0.03 |
| Comparative Example 5 | 45 | 40 | 580 | 0.7 | - |

In Table 2, the retention rate of an albumin sieving coefficient at 60 minutes after circulation start relative to an albumin sieving coefficient at 10 minutes after circulation start is described as "retention rate of albumin sieving coefficient", and the copolymer fixation amount on the inner surface of the hollow fiber membrane is described as "copolymer fixation amount".

INDUSTRIAL APPLICABILITY

The separation membrane module of the present invention has little deterioration in performance over time even when in contact with biological components such as blood for a long time, is excellent in removal performance of water and the like, and further has little amount of the eluate, and thus can be used as a separation membrane module for blood purification.

DESCRIPTION OF REFERENCE SIGNS

11: Tubular case
12: Hollow fiber membrane
13A: Header
13B: Header
14A: Blood side inlet of hollow fiber membrane
14B: Blood side outlet of hollow fiber membrane
15A: Dialysate side inlet of hollow fiber membrane
15B: Dialysate side outlet of hollow fiber membrane
16: Potting agent
17: Hollow fiber membrane module
21: Hollow fiber membrane module
22: Bi pump
23: F pump
24: Circulation beaker
25: Bi circuit
26: Bo circuit
27: F circuit
28: Heater
29: Warm water bath
31: Linear region that is produced before cantilever comes into contact with surface
32: Force curve-curved nonlinear region that appears after cantilever comes into contact with surface
33: Force curve-linear linear correlation region that appears after cantilever comes into contact with surface
34: Thickness of swelling layer
41: Membrane thickness
42: Inner diameter
43: Outer diameter

## Claims

1. A separation membrane module including a separation membrane comprising a hydrophobic polymer, a hydrophilic polymer, and polymer A,

    wherein the polymer A comprises a hydrophilic unit and a hydrophobic unit, and is a copolymer wherein the hydrophobic unit is a propyl methacrylate unit,
    and
    the separation membrane module having a retention rate of an albumin sieving coefficient of 86% or more at 60 minutes after circulation start relative to an albumin sieving coefficient at 10 minutes after circulation start when 2 L of bovine blood containing 50 U/ml of heparin, the albumin sieving coefficient being determined as specified in the description, and having a hematocrit of 30% by volume and a total protein concentration of 6 to 7 g/dl when circulated at a flow rate of 100 ml/min at 37°C and a filtration flow rate of 10 ml/(min x $m^2$).

2. The separation membrane module according to claim 1, wherein the separation membrane includes a swelling layer having a thickness of 9 to 50 nm on an inner surface of the separation membrane, and
    the inner surface of the separation membrane has a number of an adhered human platelet of 10/4.3 $\times$ $10^3$ $\mu m^2$ or less, determined as specified in the description.

3. The separation membrane module according to claim 1 or 2, wherein the separation membrane is a hollow fiber membrane having an inner diameter of 100 to 400 μm and a membrane thickness of 10 to 60 μm.

4. The separation membrane module according to claim 3, wherein the hollow fiber membrane has a total of an inner surface area of 0.3 to 3.0 $m^2$.

5. The separation membrane module according to any one of claims 1 to 4, wherein the hydrophilic unit is a vinylpyrrolidone unit, an N-vinylacetamide derivative unit, an acrylamide derivative unit, or a methacrylamide derivative unit.

6. The separation membrane module according to any one of claims 1 to 5, wherein the hydrophilic polymer is polyvinylpyrrolidone.

7. Use of the separation membrane module according to any one of claims 1 to 6 for blood purification.

## Patentansprüche

1. Trennmembranmodul, das eine Trennmembran enthält, die ein hydrophobes Polymer, ein hydrophiles Polymer und ein Polymer A umfasst, wobei das Polymer A eine hydrophile Einheit und eine hydrophobe Einheit umfasst und ein Copolymer ist, wobei die hydrophobe Einheit eine Propylmethacrylat-Einheit ist, und wobei das Trennmembranmodul eine Retentionsrate eines Albumin-Siebkoeffizienten von 86% oder mehr bei 60 Minuten nach einem Zirkulationsstart relativ zu einem Albumin-Siebkoeffizienten bei 10 Minuten nach dem Zirkulationsstart, wenn 2 l Rinderblut 50 U/ml Heparin enthalten, aufweist, wobei der Albumin-Siebkoeffizient wie in der Beschreibung spezifiziert bestimmt wird, und ein Hämatokrit von 30% des Volumens und eine Gesamtproteinkonzentration von 6 bis 7 g/dl bei einer Zirkulation mit einer Flussrate von 100 ml/min bei 37°C und einer Filtrierungsflussrate von 10 ml/(min x $m^2$) aufweist.

2. Trennmembranmodul nach Anspruch 1, wobei die Trennmembran eine Schwellschicht mit einer Dicke von 9 bis 50 nm auf einer Innenfläche der Trennmembran enthält, und die Innenfläche der Trennmembran eine Anzahl von anhaftenden menschlichen Blutplättchen von 10/4,3 $\times$ $10^3$ $\mu m^2$ oder weniger, bestimmt wie in der Beschreibung spezifiziert, aufweist.

3. Trennmembranmodul nach Anspruch 1 oder 2, wobei die Trennmembran eine Hohlfasermembran ist, die einen Innendurchmesser von 100 bis 400 μm und eine Membrandicke von 10 bis 60 μm aufweist.

4. Trennmembranmodul nach Anspruch 3, wobei die Hohlfasermembran eine Gesamtflächengröße der inneren Fläche von 0,3 bis 3,0 $m^2$ aufweist.

5. Trennmembranmodul nach einem der Ansprüche 1 bis 4, wobei die hydrophile Einheit eine Vinylpyrrolidon-Einheit, eine N-Vinylacetamid-Derivateinheit, eine Acrylamid-Derivateinheit oder eine Methacrylamid-Derivateinheit ist.

6. Trennmembranmodul nach einem der Ansprüche 1 bis 5, wobei das hydrophile Polymer Polyvinylpyrrolidon ist.

7. Verwendung des Trennmembranmoduls gemäß einem der Ansprüche 1 bis 6 für eine Blutreinigung.

## Revendications

1. Module de membrane de séparation incluant une membrane de séparation comprenant un polymère hydrophobe, un polymère hydrophile et le polymère A, dans lequel le polymère A comprend un motif hydrophile et un motif hydrophobe, et est un copolymère

   dans lequel le motif hydrophobe est un motif méthacrylate de propyle,
   et
   le module de membrane de séparation ayant un taux de rétention d'un coefficient de tamisage d'albumine de 86 % ou plus à 60 minutes après le début de la circulation par rapport à un coefficient de tamisage d'albumine à 10 minutes après le début de la circulation lorsque 2 L de sang bovin contenant 50 U/ml d'héparine ont circulé, le coefficient de tamisage d'albumine étant déterminé comme spécifié dans la description, et ayant un hématocrite

de 30 % en volume et une concentration totale en protéines de 6 à 7 g/dl lors d'une circulation à un débit de 100 ml/min à 37 °C et un débit de filtration de 10 ml/(min x $m^2$).

**2.** Module de membrane de séparation selon la revendication 1, dans lequel la membrane de séparation inclut une couche de gonflement ayant une épaisseur de 9 à 50 nm sur une surface interne de la membrane de séparation, et la surface interne de la membrane séparation a un nombre de plaquettes humaines ayant adhéré de 10/4,3 x $10^3$ $\mu m^2$ ou moins, déterminé comme spécifié dans la description.

**3.** Module de membrane de séparation selon la revendication 1 ou 2, dans lequel la membrane de séparation est une membrane à fibres creuses ayant un diamètre interne de 100 à 400 $\mu$m et une épaisseur de membrane de 10 à 60 $\mu$m.

**4.** Module de membrane de séparation selon la revendication 3, dans lequel la membrane à fibres creuses a une aire interne totale de 0,3 à 3,0 $m^2$.

**5.** Module de membrane de séparation selon l'une quelconque des revendications 1 à 4, dans lequel le motif hydrophile est un motif vinylpyrrolidone, un motif dérivé de N-vinylacétamide, un motif dérivé d'acrylamide ou un motif dérivé de méthacrylamide.

**6.** Module de membrane de séparation selon l'une quelconque des revendications 1 à 5, dans lequel le polymère hydrophile est la polyvinylpyrrolidone.

**7.** Utilisation du module de membrane de séparation selon l'une quelconque des revendications 1 à 6 pour la purification du sang.

[Fig 1]

[Fig 1]

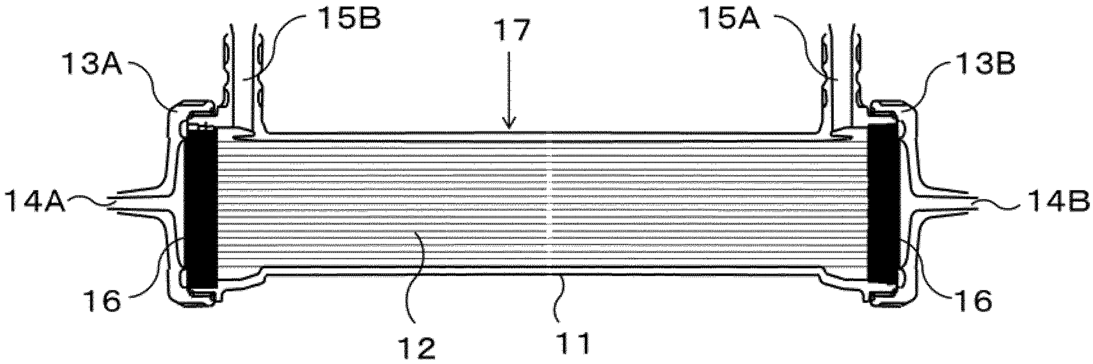


[Fig 2]

[Fig 2]

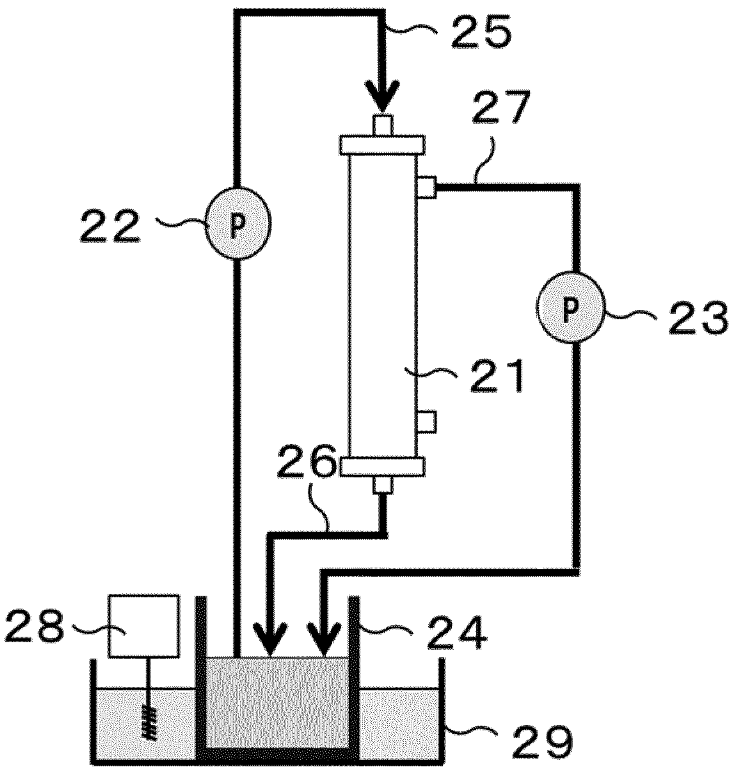

[Fig 3]

[Fig 3]

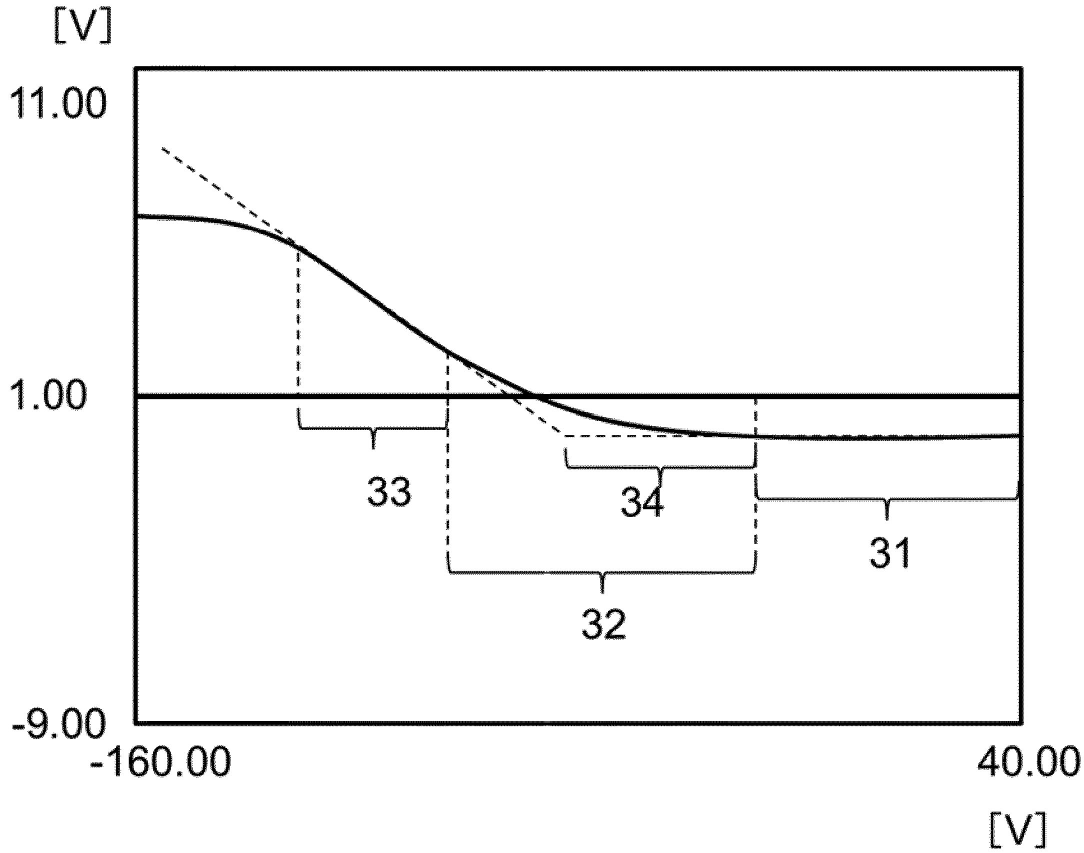


[Fig 4]

[Fig 4]

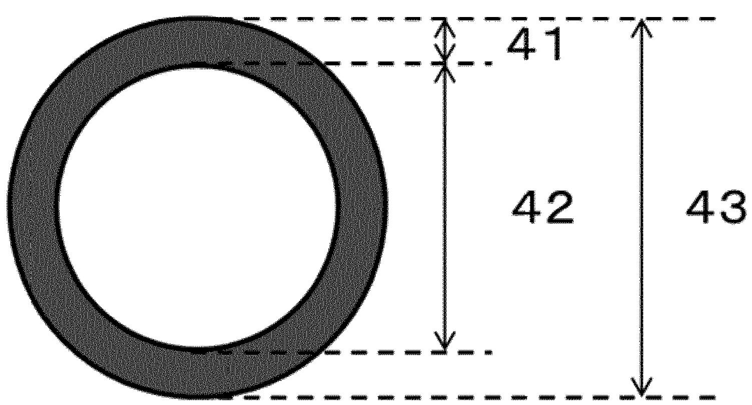

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2737916 A
- JP 2018695 B
- JP 6238139 A
- JP 2010104984 A
- JP 2011173115 A
- JP 2006198611 A